# EUROPEAN PATENT SPECIFICATION

(11) **EP 3 494 210 B1**
(45) Date of publication and mention of the grant of the patent: **09.10.2024**
(21) Application number: 17837822.0
(22) Date of filing: 07.08.2017
(51) Int. Cl.: C12Q 1/6883

(54) **DNA METHYLATION BASED PREDICTOR OF MORTALITY**
AUF DNA-METHYLIERUNG BASIERENDER PRÄDIKTOR FÜR DIE STERBLICHKEIT
PRONOSTIQUEUR DE MORTALITÉ BASÉ SUR LA MÉTHYLATION DE L'ADN

(30) Priority: 05.08.2016 US 201662371624 P
(43) Date of publication of application: 12.06.2019
(73) Proprietor: THE REGENTS OF THE UNIVERSITY OF CALIFORNIA, Oakland, CA 94607-5200 (US)
(72) Inventor: HORVATH, Stefan, Los Angeles, California 90049 (US)
(74) Representative: J A Kemp LLP
(86) International application number: PCT/US2017/045764
(87) International publication number: WO 2018/027228

(56) References cited:
- WO-A1-2011/135088
- WO-A2-2015/048665
- C. FUKE ET AL: "Age Related Changes in 5-methylcytosine Content in Human Peripheral Leukocytes and Placentas: an HPLC-based Study", ANNALS OF HUMAN GENETICS, vol. 68, no. 3, 1 May 2004 (2004-05-01), pages 196 - 204, XP055055345, ISSN: 0003-4800, DOI: 10.1046/j.1529-8817.2004.00081.x
- BOCKLANDT S ET AL: "Epigenetic Predictor of Age", PLOS ONE,, vol. 6, no. 6, 22 June 2011 (2011-06-22), pages E14821 - 1, XP002687317, ISSN: 1932-6203, DOI: 10.1371/JOURNAL.PONE.0014821
- NAUE JANA ET AL: "Chronological age prediction based on DNA methylation: Massive parallel sequencing and random forest regression", FORENSIC SCIENCE INTERNATIONAL: GENETICS, vol. 31, 1 August 2017 (2017-08-01), pages 19 - 28, XP085298174, ISSN: 1872-4973, DOI: 10.1016/J.FSIGEN.2017.07.015
- GREGORY HANNUM ET AL: "Genome-wide Methylation Profiles Reveal Quantitative Views of Human Aging Rates", MOLECULAR CELL, vol. 49, no. 2, 1 January 2013 (2013-01-01), pages 359 - 367, XP055160619, ISSN: 1097-2765, DOI: 10.1016/j.molcel.2012.10.016
- STEVE HORVATH: "DNA methylation age of human tissues and cell types", GENOME BIOLOGY, BIOMED CENTRAL LTD, vol. 14, no. 10, 21 October 2013 (2013-10-21), pages R115, XP021165700, ISSN: 1465-6906, DOI: 10.1186/GB-2013-14-10-R115
- HORVATH ET AL.: "HIV-1 Infection Accelerates Age According to the Epigenetic Clock", J INFECT DIS, vol. 212, no. 10, 15 November 2015 (2015-11-15), pages 1563 - 1573, XP055461073
- DAY ET AL.: "Differential DNA methylation with age displays both common and dynamic features across human tissues that are influenced by CpG landscape", GENOME BIOL, vol. 14, no. 9, 13 September 2013 (2013-09-13), pages 1 - 19, XP021165718

## Description

### TECHNICAL FIELD

The invention relates to epigenetics and in particular, methods of determining the epigenetic aging of an individual and predicting mortality based on DNA methylation-based biomarkers, as defined in the appended claims.

### BACKGROUND OF THE INVENTION

Recently developed DNA methylation-based biomarkers allow one to estimate the epigenetic age of an individual [1-4]. For example, the "epigenetic clock" which is based on 353 dinucleotide markers, known as CpGs (-C-phosphate-G-), can be used to estimate the age of most human cell types, tissues, and organs [3]. The estimated age, referred to as "DNA methylation age" (DNAm age), correlates with chronological age when methylation is assessed in sorted cell types (CD4+ T cells, monocytes, B cells, glial cells, neurons), tissues, and organs including whole blood, brain, breast, kidney, liver, lung, and saliva [3]. Other reports have described DNAm-based biomarkers that pertain to a single tissue (e.g. saliva or blood) [1-2, 4, 36]. Recent studies have suggested that DNAm-based biomarkers of age capture aspects of biological age. For example, studies have previously shown that individuals whose DNAm age was greater than their chronological age, i.e. individuals who exhibited epigenetic "age acceleration", were at an increased risk for death from all causes even after accounting for known risk factors [5-7]. Epigenetic age acceleration is sometimes referred to as epigenetic aging rate. Further, it has been recently shown that the offspring of semi-supercentenarians (individuals who reached an age of 105-109 years) have a lower epigenetic age than age-matched controls [8]. These results demonstrate that epigenetic data give risk to epigenetic biomarkers of mortality (referred to as biological age estimates).

Accordingly, there is a need for methods of determining biological age, epigenetic age acceleration/aging rate, which is predictive of an earlier age of death (all-cause mortality) that is independent of chronological age and traditional risk factors of mortality.

### SUMMARY OF THE INVENTION

Estimates of age based on DNA methylation patterns, often referred to as "epigenetic age" or "DNAm age", have been shown to be robust biomarkers of age in humans. As disclosed herein, it has been discovered that incorporating information on blood cell composition into the DNAm age estimates further improves their predictive power for mortality. In this context, systems and methods are provided herein for examining blood cell counts and determining the biological age and epigenetic age acceleration of an individual or biological sample. Such systems and methods are able to predict all-cause mortality of an individual above and beyond chronological age and traditional risk factors. By estimating blood cell counts, the invention described herein greatly can enhance conventional DNA methylation based biomarker observations of aging based on 71 CpGs such as those described in Hannum et al. 2013 MolCell. 2013 Jan 24; 49(2):359-67, PMID: 23177740. The resulting biological age estimate and related measures of epigenetic age acceleration obtained by embodiments of the invention are significantly better predictors of mortality than the original method developed by Hannum (see, also U.S. Patent Publication 20150259742).

Herein described is a method for determining the epigenetic age acceleration/aging rate of an individual. The method comprises measuring a methylation level of a set of methylation markers that allow one to estimate certain cell types in an individual and determining the biological age of the individual based on the measured methylation level. For example, the biological age of the individual can be determined using a weighted average of the DNA methylation levels to observe the DNA methylation age, and blood cell counts of naive cytotoxic T cells, exhausted cytotoxic T cells, and/or plasma B cells in the individual, specific cell counts that can be used to provide information on the biological age of the individual. The determined biological age is then compared to a chronological age of the individual to determine an epigenetic age acceleration of the individual. Instances wherein the observed epigenetic age is greater than expected based on the chronological age of the individual is an indication of an increased risk of all-cause mortality.

In one or more embodiments, the biological age of the individual is estimated using a methylation based weighted average to determine counts (or relative abundances) of naive cytotoxic T cells, exhausted cytotoxic T cells, plasma B cells, and DNA methylation age in the individual. The estimate of the epigenetic age can comprise applying weighted averages of methylation patterns observed to obtain information on the naive cytotoxic T cells, exhausted cytotoxic T cells, and/or plasma B cells and then age, in view of a correlation between the counts of naive cytotoxic T cells, exhausted cytotoxic T cells, and/or plasma B cells and the chronological age of the individual. In this context, epigenetic age acceleration is positively correlated with the cell count of the exhausted cytotoxic T cells and plasma B cells and is negatively correlated with the cell count of the naive cytotoxic T cells. In typical embodiments, the biological age of the individual is determined by (a) obtaining a linear combination of the methylation marker levels, and (b) applying a transformation to the linear combination to determine the biological age of the individual.

In another embodiment of the invention, a method for observing the health of an individual is provided. The method comprises collecting a tissue sample from an individual and extracting genomic DNA from the collected tissue sample. Methylation of a plurality of methylation marker on the genomic DNA is then observed and a biological age estimate of the individual is then determined with a statistical prediction algorithm. Typically, the statistical prediction algorithm is applied to the measured methylation of key methylation markers. A biological age estimate of the individual is determined based on a weighted average of the DNA methylation markers/levels to observe cell counts of naive cytotoxic T cells, exhausted cytotoxic T cells, and the DNAm age in the individual. The determined biological age is then compared to a chronological age of the individual. Instances where the epigenetic age is greater than expected based on the chronological age of the individual is an indication of a higher mortality risk of the individual compared to other people of the same chronological age.

In another embodiment of the invention, a tangible computer-readable medium is provided comprising computer-readable code that, when executed by a computer, causes the computer to perform operations comprising receiving information corresponding to methylation levels of a set of methylation markers in a biological sample. The computer-readable code further causes the computer to determine a biological age estimate by applying a statistical prediction algorithm to the set of methylation marker, determine a biological age estimate based on weighted cell counts of plasm B cells, naive cytotoxic T cells, exhausted cytotoxic T cells, and DNAm age, and compare the determined biological age to a chronological age of the biological sample. In certain instances, the step of receiving information comprises receiving from a tangible data storage device information corresponding to the methylation levels of the set of methylation markers in the biological sample. The tangible computer-readable medium may further comprise computer-readable code that, when executed by a computer, causes the computer to send information corresponding to the methylation levels of the set of methylation markers in the biological sample to a tangible data storage device.

### BRIEF DESCRIPTION OF THE DRAWINGS

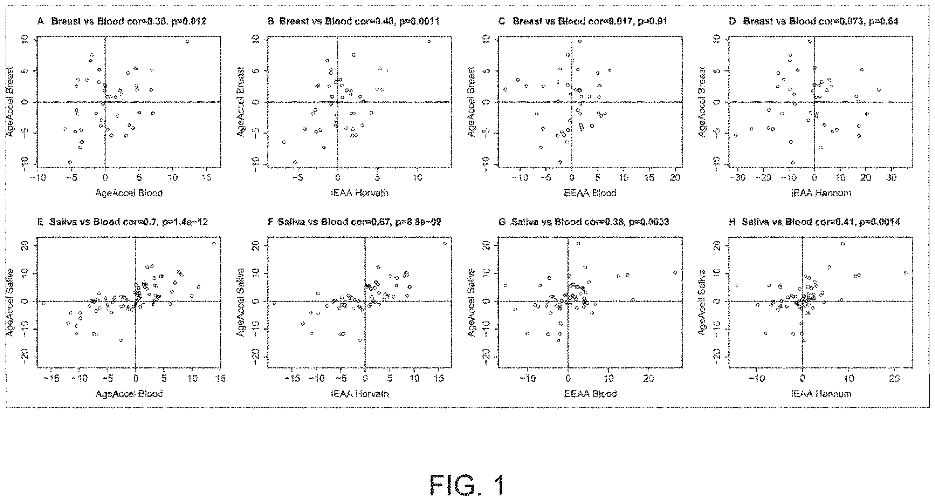

Figure 1. Epigenetic age acceleration in blood versus that in breast or saliva. A-D) Epigenetic age acceleration in healthy female breast tissue (y-axis) versus various measures of epigenetic age acceleration in blood: A) universal measure of age acceleration in blood, B) intrinsic epigenetic age acceleration based on the Horvath estimate of DNA methylation age, C) extrinsic epigenetic age acceleration, D) intrinsic epigenetic age acceleration based on the Hannum estimate of DNA methylation age. E-H) analogous plots for epigenetic age acceleration in saliva (y-axis) and E) *AgeAccel,* F) *IEAA* based on Horvath, G) *EEAA,* H) *IEAA* based on the Hannum estimate. The y-axis of each plot represents the universal measure of age acceleration defined as the raw residual resulting from regressing DNA methylation age (based on Horvath) on chronological age.

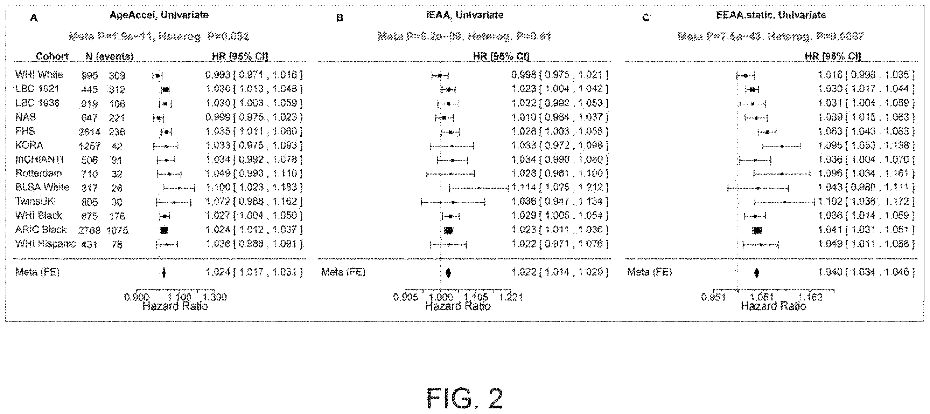

Figure 2. Univariate Cox regression meta-analysis of all-cause mortality. A univariate Cox regression model was used to relate the censored survival time (time to all-cause mortality) to A) the universal measure of age acceleration *(AgeAccel),* B) intrinsic accelerated epigenetic age *(IEAA),* C) extrinsic accelerated epigenetic age *(EEAA).* The rows correspond to the different cohorts. Each row depicts the hazard ratio and a 95% confidence interval. The coefficient estimates from the respective studies were meta-analyzed using a fixed-effect model weighted by inverse variance (implemented in the *metafor* R package [32]). It is not appropriate to compare the hazard ratios and confidence intervals of the different measures directly because the measures have different scales/distributions. However, it is appropriate to compare the meta analysis p values (red sub-title of each plot). The p-value of the heterogeneity test (Cochran's *Q*-test) is significant if the cohort-specific estimates differed substantially.

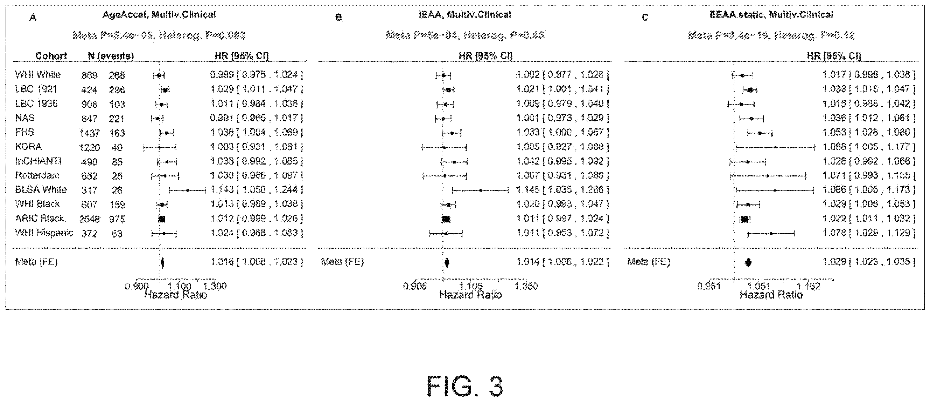

Figure 3. Multivariate Cox regression meta-analysis adjusted for clinical covariates. A multivariate Cox regression model was used to relate the censored survival time (time to all-cause mortality) to A) the universal measure of age acceleration *(AgeAccel),* B) intrinsic epigenetic age acceleration *(IEAA),* C) extrinsic epigenetic age acceleration *(EEAA).* The multivariate Cox regression model included the following additional covariates: chronological age, body mass index (category), educational level (category), alcohol intake, smoking pack years, prior history of diabetes, prior history of cancer, hypertension status, recreational physical activity (category). The rows correspond to the different cohorts. Each row depicts the hazard ratio and a 95% confidence interval. The coefficient estimates from the respective studies were meta-analyzed using a fixed-effect model weighted by inverse variance (implemented in the *metafor* R package [32] ). The sub-title of each plot reports the meta-analysis p-value and a heterogeneity test p-value (Cochran's Q-test).

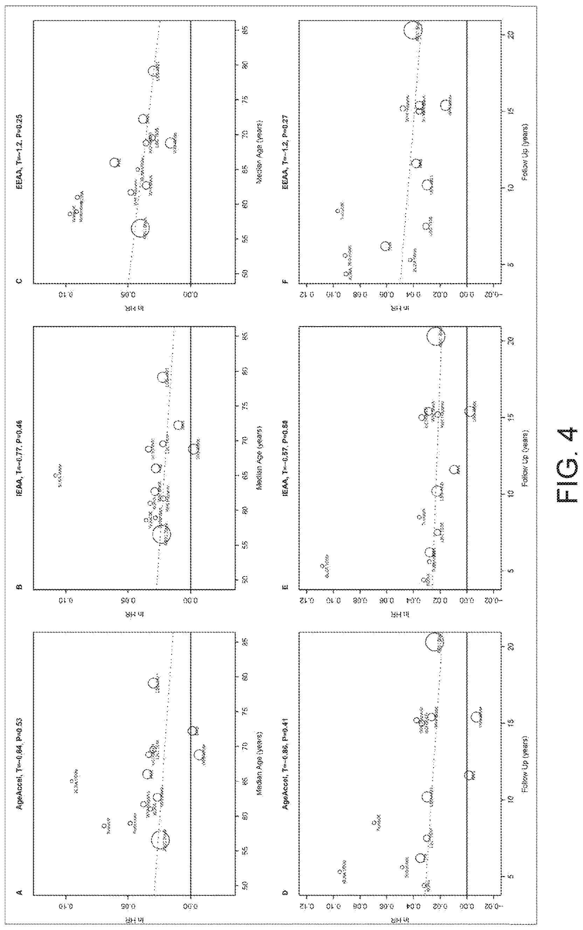

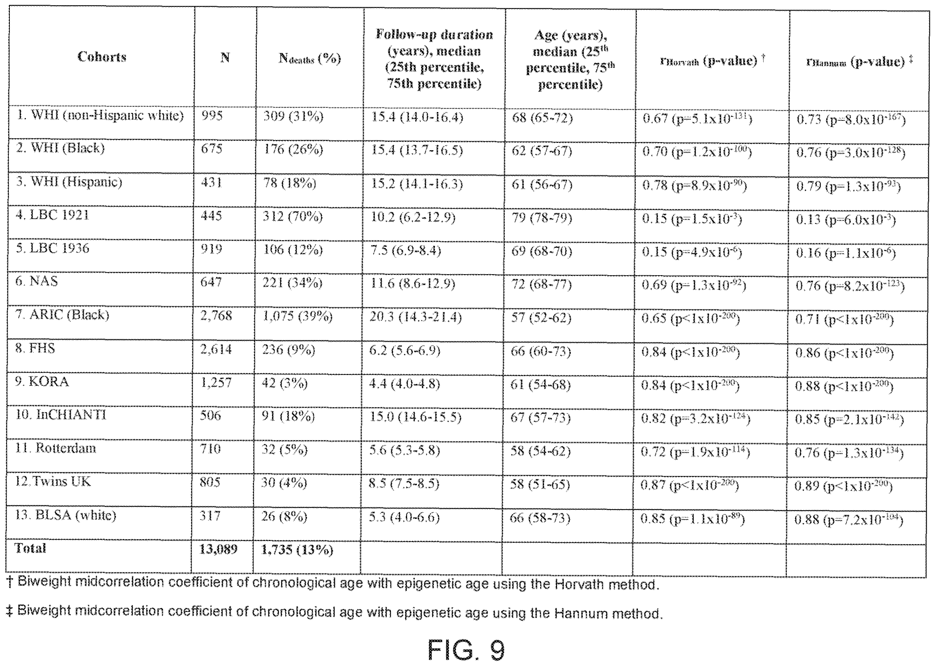

Figure 4. Hazard ratio of death versus cohort characteristics. Each circle corresponds to a cohort (data set). Circle sizes correspond to the square root of the number of observed deaths, because the statistical power of a Cox model is determined by the number of observed deaths. A-C) The y-axis of each panel corresponds to the natural log of the hazard ratio (ln HR) of a univariate Cox regression model for all-cause mortality. Each panel corresponds to a different measure of epigenetic age acceleration A) universal age acceleration, B) intrinsic age acceleration, C) extrinsic age acceleration. Panels D-F are analogous to those in A-C but the x-axis corresponds to the median age of the individuals at baseline (Figure 9). The title of each panel reports the Wald test statistic (*T*) and corresponding p-value resulting from a weighted linear regression model (y regressed on x) where each point (data set) is weighted by the square root of the number of observed deaths. The dotted red line represents the regression line. The black solid line represents the line of identify (i.e., no association).

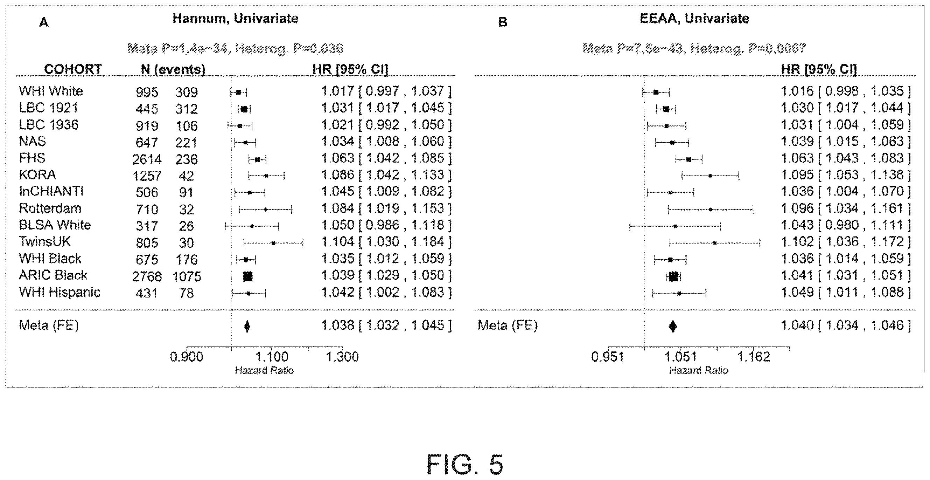

Figure 5. Univariate Cox regression model analysis of all cause mortality, contrasting existing with novel measures of age acceleration. The rows correspond to the different cohorts. Each row depicts the hazard ratio and a 95% confidence interval. To combine the coefficient estimates from the respective studies into a single estimate, we applied a fixed- effect model weighted by inverse variance (implemented in the "metafor" R package [30]). A) This measure of age acceleration is based on Hannum et al [2]. Specifically, we estimated the age using the 71 CpGs and coefficient values from Hannum. Next, the measure of age acceleration was defined as residuals resulting from regressing the age estimate on chronological age. B) Extrinsic epigenetic age acceleration. The sub-title of each plot reports the meta-analysis p-value and a heterogeneity test p-value (Cochran's Q-test).

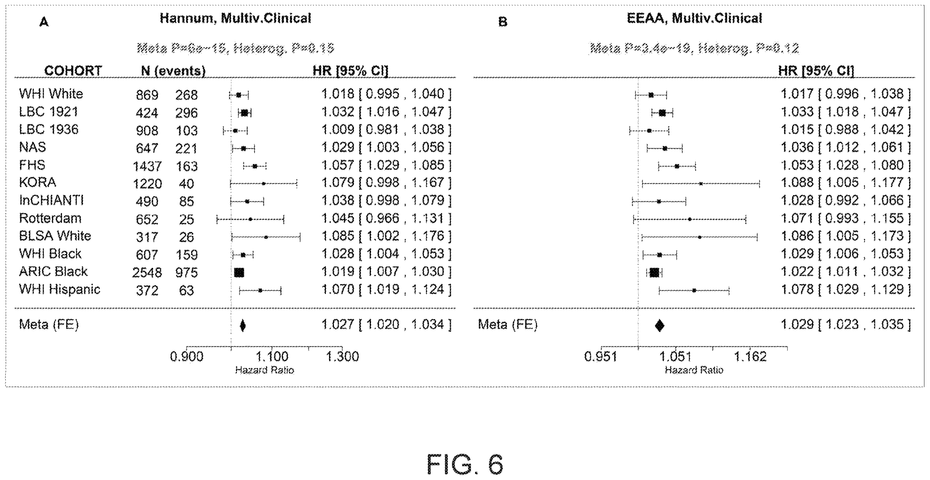

Figure 6. Multivariate Cox regression model analysis of all-cause mortality, contrasting existing with novel measures of age acceleration. The multivariate Cox regression model included the following additional covariates: chronological age, body mass index (category), educational level (category), alcohol intake, smoking pack years, prior history of diabetes, prior history of cancer, hypertension status, recreational physical activity (category). The rows correspond to the different cohorts. Each row depicts the hazard ratio and a 95% confidence interval. A) Age acceleration based on Hannum et al [6]. B) Extrinsic epigenetic age acceleration. The sub-title of each plot reports the meta-analysis p-value and a heterogeneity test p-value (Cochran's Q-test).

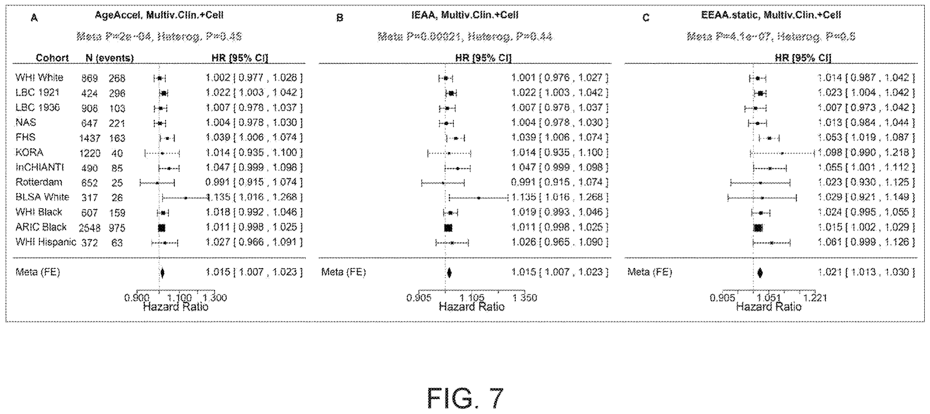

Figure 7. Multivariate Cox regression analysis of all cause mortality that adjusted for blood cell counts and clinical covariates. A multivariate Cox regression model was used to relate the censored survival time (time to all-cause mortality) to A) the universal measure of age acceleration (AgeAccel), B) intrinsic epigenetic age acceleration *(IEAA),* C) extrinsic epigenetic age acceleration *(EEAA).* The multivariate Cox regression model included blood cell counts (exhausted CD8+ T cells, naive CD8+, CD4+ T cells, natural killer, monocytes, granulocytes, and plasma B cells) and clinical covariates (chronological age, body mass index, educational level, alcohol intake, smoking pack years, prior history of diabetes, prior history of cancer, hypertension status, recreational physical activity). The rows correspond to the different cohorts. Each row depicts the hazard ratio and a 95% confidence interval. To combine the coefficient estimates from the respective studies into a single estimate, we applied a fixed-effect model weighted by inverse variance. The sub-title of each plot reports the meta-analysis p-value and a heterogeneity test p-value (Cochran's Q-test).

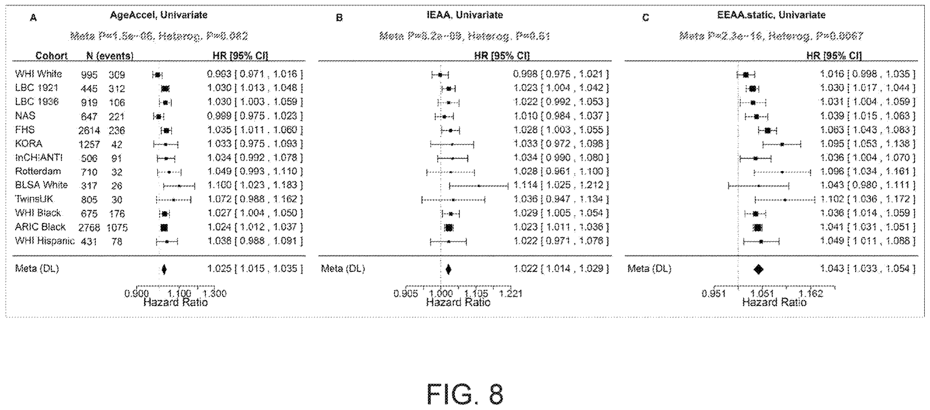

Figure 8. Random effects meta analysis for univariate Cox models. The figure is analogous to Figure 2 except that it uses a random effects meta analysis (DerSimion Laird) instead of a fixed effects model. A univariate Cox regression model was used to relate the censored survival time (time to all-cause mortality) to A) the universal measure of age acceleration (AgeAccel), B) intrinsic epigenetic age acceleration *(IEAA)*, C) extrinsic epigenetic age acceleration *(EEAA).* To combine the coefficient estimates from the respective studies into a single estimate, the DerSimion-Laird random effects model was applied. The sub-title of each plot reports the meta-analysis p-value and a heterogeneity test p-value (Cochran's Q-test).
Figure 9. Baseline characteristics of participating cohorts. The last 3 columns report robust correlation coefficients (biweight midcorrelation) between chronological age and two DNA methylation age estimates (Horvath and Hannum).

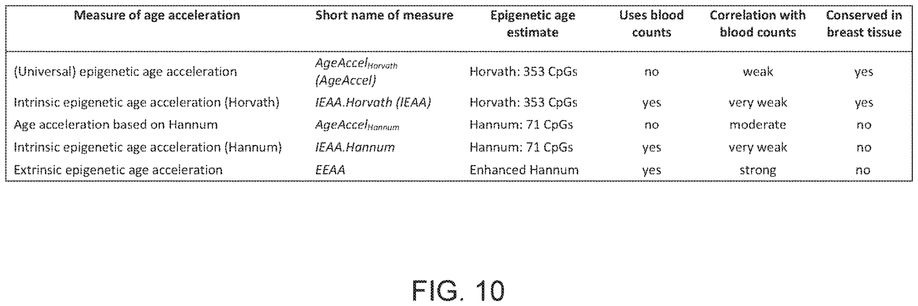

Figure 10. Age acceleration definitions. Description of the differences between DNA methylation age and age acceleration measures.

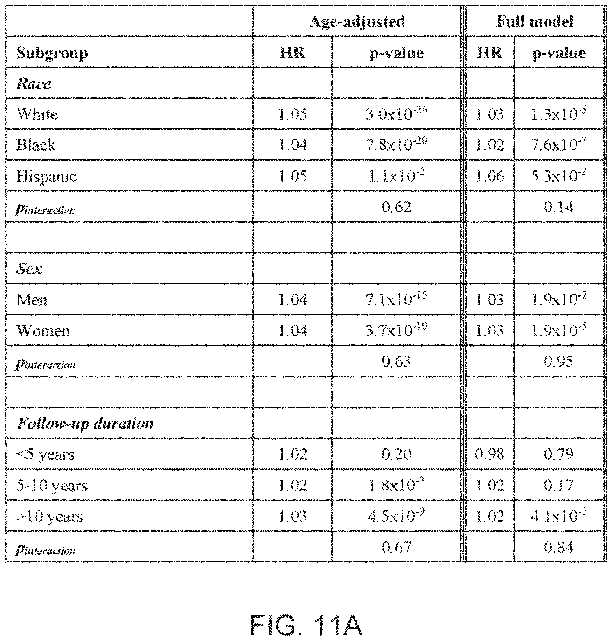

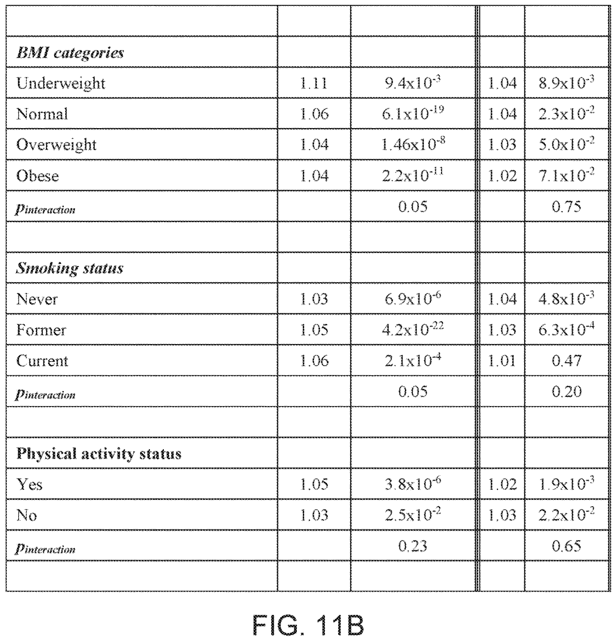

Figures 11A-B. Subgroup analysis by demographic factors. Age-adjusted and fully adjusted associations for *EEAA* to all-cause mortality by subgroup (rows). The fully adjusted model includes the following covariates: body mass index, educational level, alcohol intake, smoking pack-years, prior history of diabetes, prior history of cancer, hypertension status, self-reported recreational physical activity.

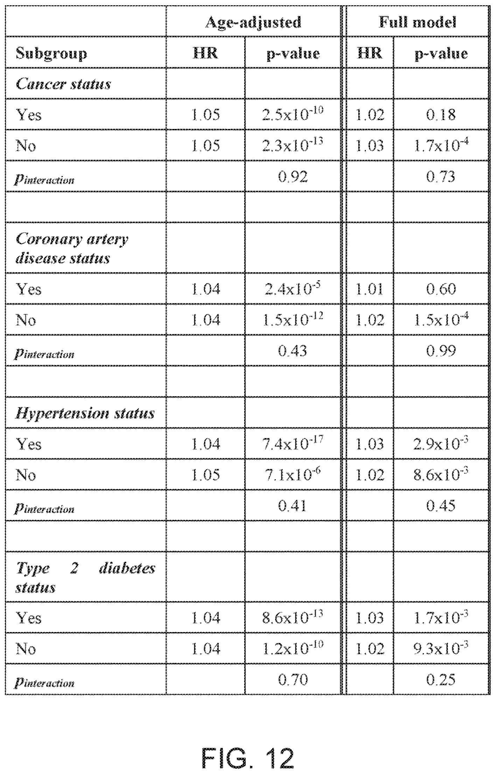

Figure 12. Subgroup analysis by prevalent disease status. Age-adjusted and fully adjusted associations for *EEAA* to all-cause mortality in different subgroups (rows). The fully adjusted model includes the following covariates: body mass index, educational level, alcohol intake, smoking pack-years, prior history of diabetes, prior history of cancer, hypertension status, self-reported recreational physical activity.

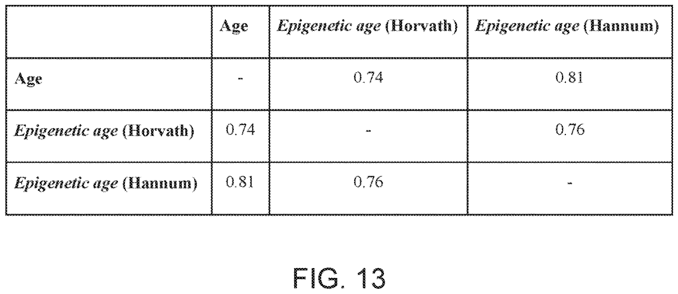

Figure 13. Average pairwise correlations between chronological age, DNAm age based on the Horvath method, and DNAm age based on Hannum.

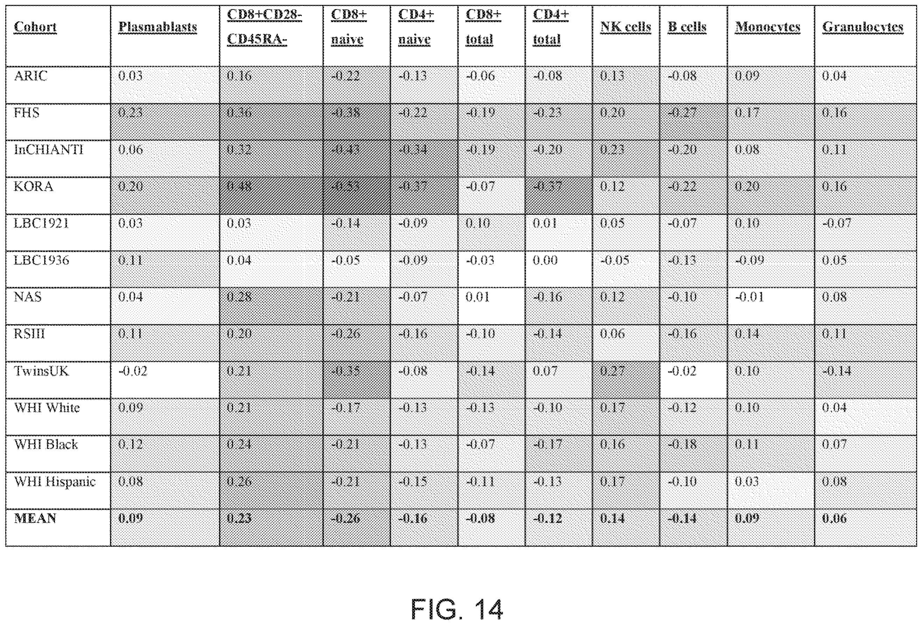

Figure 14. Correlations between estimated blood cell abundances and chronological age. The values shown are robust correlation coefficients (biweight midcorrelation, which is based on medians). Colors reflect the direction and magnitude of the correlation coefficients (blue=negative correlation, red=positive correlation). The extrinsic age estimate (denoted BioAge4HA in our software) gives rise to the measure of extrinsic age acceleration.

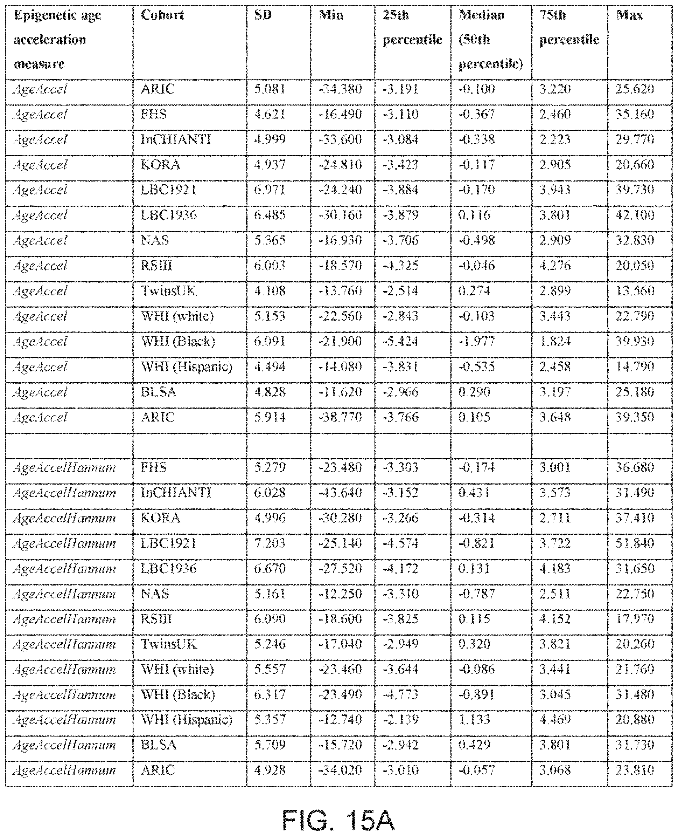

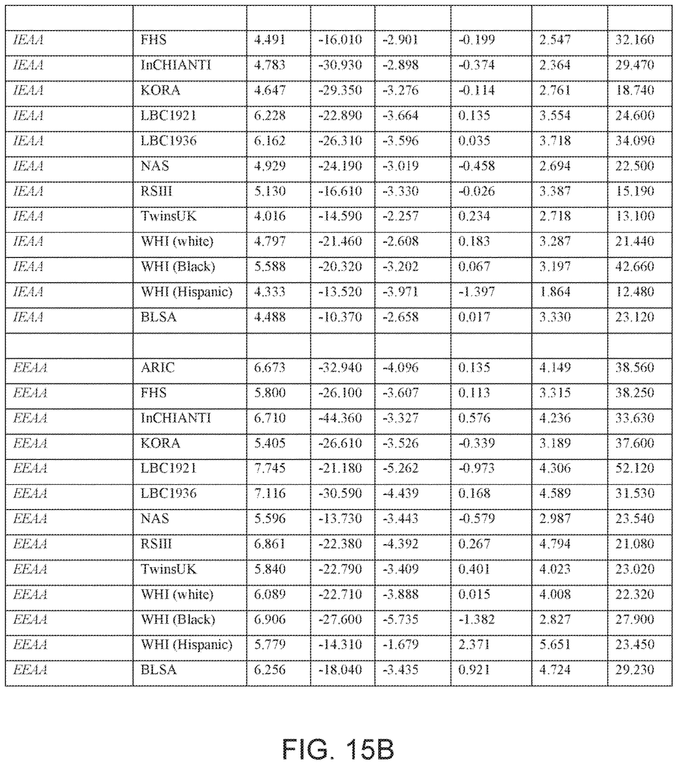

Figures 15A-B. Descriptive statistics of measures of epigenetic age acceleration by cohort.

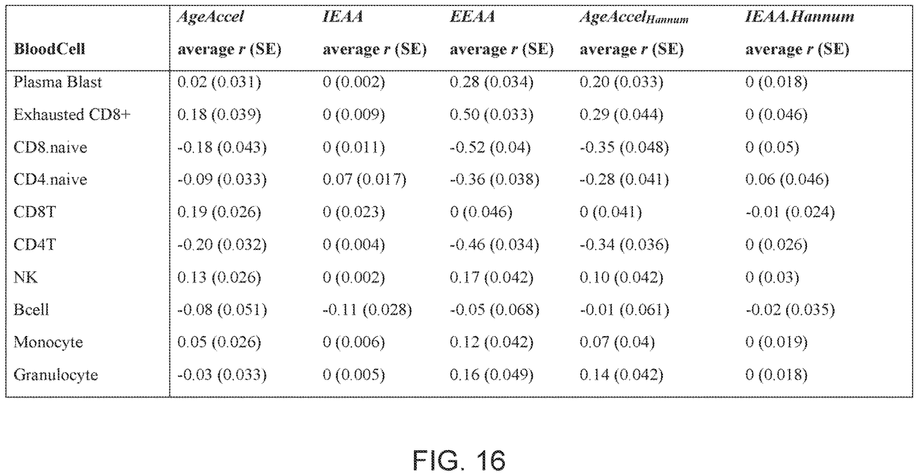

Figure 16. Pairwise correlations (mean and standard error (SE) across cohorts) between blood cell counts estimated from DNA methylation profiles (rows) and several measures of epigenetic age acceleration (columns). *AgeAccel=universal* measure of age acceleration based on Horvath estimate. *IEAA*=intrinsic epigenetic age acceleration based on the Horvath estimate. *EEAA* = extrinsic epigenetic age acceleration which is an enhanced version of the Hannum estimate. *AgeAccel_{Hannum}*=universal measure of age acceleration based on the Hannum estimate. *IEAA.Hannum=intrinsic* epigenetic age acceleration based on Hannum estimate. By design, the intrinsic measures have only weak correlations with blood cell counts. By contrast, *AgeAccel_{Hannum}* and *EEAA* have moderately strong correlations with blood cell counts.

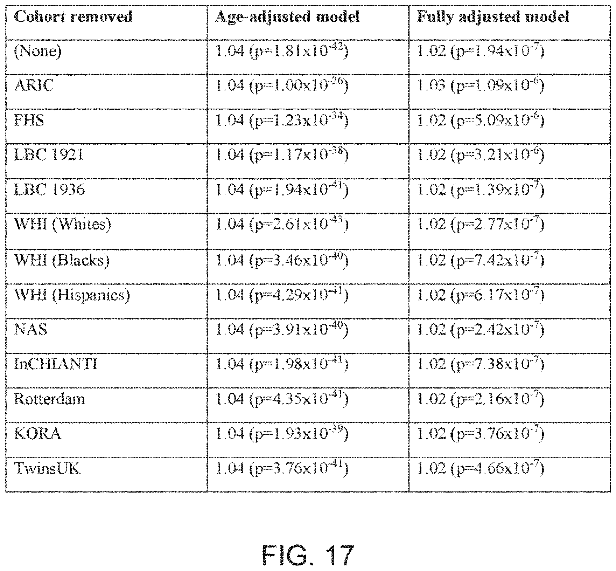

Figure 17. Leave-one-out analysis by cohort for relating *EEAA* to time to death. The table reports hazards ratios and corresponding p-values based on a Cox regression. The fully adjusted model includes the following covariates: body mass index, educational level, alcohol intake, smoking pack-years, prior history of diabetes, prior history of cancer, hypertension status, self-reported recreational physical activity.

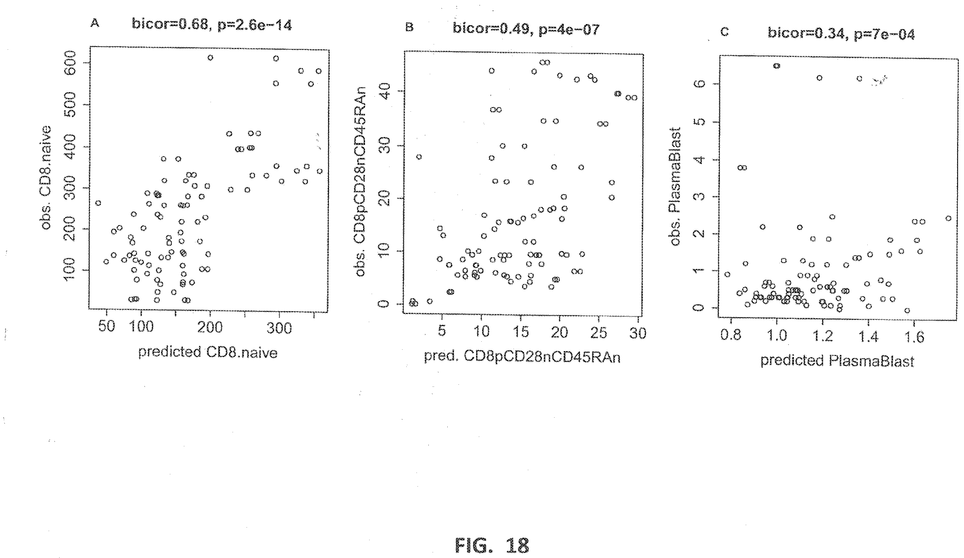

Figure 18. Observed flow cytometric measures of blood cell counts versus estimated blood cell counts based on DNA methylation levels. A) The predicted/estimated count of naive CD8+ T cells (x-axis) is highly correlated with the observed count based on flow cytometric measures. B) Analogous graph for the percentage of exhausted CD8+ T cells defined as CD28 negative, CD45RA negative markers. C) Analogous plot for percentage of plasma blasts. The flow cytometric data and DNA methylation data were assessed in 96 individuals between 30 and 70 years of age who were not used in the development of the method, i.e. they represent an unbiased validation set.

### DETAILED DESCRIPTION OF THE INVENTION

Many of the techniques and procedures described or referenced herein are well understood and commonly employed by those skilled in the art. Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art.

All publications mentioned herein disclose and describe aspects, methods and/or materials in connection with the cited publications. For example, U.S. Patent Publication 20150259742, and Hannum et al. "Genome-Wide Methylation Profiles Reveal Quantitative Views Of Human Aging Rates." Molecular Cell. 2013; 49(2) 359-367 and patent US20150259742. Furthemore Fuke et al., Annals of Human Genetics, 68,pp.196-204,2004; Naue et al., Forens. Sci. Int., 31, pp.19-28,2017; WO2011/135088 A1 describe the use of methylation pattern for evaluating age.

Novel biomarkers of aging, such as those termed "biological age", "epigenetic age" or "apparent methylomic aging rate" and "DNAm" herein are assessments of physiological profiles in individuals that allow one to prognosticate mortality, are interesting to medical underwriters of life insurance products. It is useful to distinguish clinical from molecular biomarkers of aging. Clinical biomarkers such as lipid levels, body mass index, blood pressures have a long and successful history in the life insurance industry. By contrast, molecular biomarkers of aging are rarely used. However, this is likely to change due to recent breakthroughs in the development of molecular biomarkers of aging.

The profitability of a life insurance product directly depends on the accurate assessment of mortality risk because the costs of life insurance (to the insurance company) are directly proportional to the number of deaths in a given category. Thus, any improvement in assessing mortality risk and in improving the basic classification will directly translate into cost savings. For the reasons noted above, DNA methylation (DNAm) based biomarkers of aging are useful for predicting mortality. Consequently, they are useful the life insurance industry due to their ability to increase the accuracy of medical underwriting. DNAm measurements can provide a host of complementary information that can inform the medical underwriting process. In this context, the DNAm based biomarkers and associated method disclosed herein can be used both to molecularly estimate complete blood counts and to estimate biological age, as well as to directly predict/prognosticate mortality. Using embodiments of the invention disclosed herein, upon completing a medical exam, an insurer can, for example, look at the DNA methylation test results as well as other factors such as family health history and lifestyle choices to classify the applicant into useful classification categories such as: 1) preferred plus/super preferred/preferred select/preferred elite, 2) preferred, 3) standard plus, 4) standard, 5) preferred smoker, 6) standard smoker, 7) table rate A, 8) table rate B, etc. Each of these categories has a distinct mortality risk and usually directly relates to the pricing of the insurance product. The basic classification is largely determined by well established risk factors of mortality such as sex, smoking status, family history of death, prior history of disease (e.g. diabetes status, cancer), and a host of clinical biomarkers (blood pressure, body mass index, cholesterol, glucose levels, hemoglobin A1C).

DNA methylation refers to chemical modifications of the DNA molecule. Technological platforms such as the Illumina Infinium microarray or DNA sequencing based methods have been found to lead to highly robust and reproducible measurements of the DNA methylation levels of a person. While the DNA molecule contains millions of sites (known as CpGs) that can be methylated, relatively few such sites are needed to inform the risk assessment process. For example, only the 143 CpGs sites disclosed in TABLE 1 herein are needed for estimating the biological age. The 143 measurements are combined into a single number (referred to herein as "biological age" or at times, BioAge4). This biological age estimate or BioAge4 is a number in units of years (e.g. 60 years) that is usually distinct from the chronological age (e.g. 50 years) of a person. The difference in BioAge4 and chronological age (e.g. 10 years) has been found to be prognostic of life span (all cause mortality).

The term "epigenetic" as used herein means relating to, being, or involving a chemical modification of the DNA molecule. Epigenetic factors include the addition or removal of a methyl group which results in changes of the DNA methylation levels.

The term "nucleic acids" as used herein may include any polymer or oligomer of pyrimidine and purine bases, preferably cytosine, thymine, and uracil, and adenine and guanine, respectively. The present invention contemplates any deoxyribonucleotide, ribonucleotide or peptide nucleic acid component, and any chemical variants thereof, such as methylated, hydroxymethylated or glucosylated forms of these bases, and the like. The polymers or oligomers may be heterogeneous or homogeneous in composition, and may be isolated from naturally-occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states.

The term "methylation marker" as used herein refers to a CpG position that is potentially methylated. Methylation typically occurs in a CpG containing nucleic acid. The CpG containing nucleic acid may be present in, e.g., in a CpG island, a CpG doublet, a promoter, an intron, or an exon of gene. For instance, in the genetic regions provided herein the potential methylation sites encompass the promoter/enhancer regions of the indicated genes. Thus, the regions can begin upstream of a gene promoter and extend downstream into the transcribed region.

The term "gene" as used herein refers to a region of genomic DNA associated with a given gene. For example, the region can be defined by a particular gene (such as protein coding sequence exons, intervening introns and associated expression control sequences) and its flanking sequence. It is, however, recognized in the art that methylation in a particular region is generally indicative of the methylation status at proximal genomic sites. Accordingly, determining a methylation status of a gene region can comprise determining a methylation status of a methylation marker within or flanking about 10 bp to 50 bp, about 50 to 100 bp, about 100 bp to 200 bp, about 200 bp to 300 bp, about 300 to 400 bp, about 400 bp to 500 bp, about 500 bp to 600 bp, about 600 to 700 bp, about 700 bp to 800 bp, about 800 to 900 bp, 900 bp to 1kb, about 1 kb to 2 kb, about 2 kb to 5 kb, or more of a named gene, or CpG position.

The phrase "selectively measuring" as used herein refers to methods wherein only a finite number of methylation marker or genes (comprising methylation markers) are measured rather than assaying essentially all potential methylation marker (or genes) in a genome. For example, in some aspects, "selectively measuring" methylation markers or genes comprising such markers can refer to measuring no more than 100, 75, 50, 25, 10 or 5 different methylation markers or genes comprising methylation markers.

As described above, recently developed DNA methylation-based biomarkers allow one to estimate the epigenetic age of an individual [1-4]. For example, the "epigenetic clock" which is based on methylation levels of dinucleotide markers, known as CpGs (-C-phosphate-G-), can be used to estimate the age of most human cell types, tissues, and organs [3]. The estimated age, referred to as "DNA methylation age" (DNAm age) is highly correlated with chronological age. However, DNAm age can deviate substantially from chronological age. Deviations between DNAm age and chronological age can be used to define "delta age" and various measures of epigenetic "age acceleration". For example, one such measure (denoted as AgeAccel) is defined as the residual that results from regressing DNAm age on chronological age. Thus, a positive value of AgeAccel indicates that the epigenetic age is higher than expected based on chronological age.

The present invention pertains to a novel measure of epigenetic age acceleration which is predictive of earlier age at death (all-cause mortality) independent of chronological age and traditional risk factors of mortality. Several previous measures of age acceleration (denoted as AgeAccelHannum and AgeAccelHorvath) were previously shown to correlate with blood cell counts [3]. The fact that the Hannum measure of age acceleration (AgeAccelHannum) is confounded by blood cell counts that change with age can be an advantageous property because this measure might capture aspects of immunosenescence. The invention amplifies this property by using an enhanced measure of extrinsic age acceleration *(EEAA)* that is even more associated with blood cell counts than the original Hannum measure. This novel measure of *EEAA* results from a weighted average of the Hannum estimate with three measures of blood cell types that are known to change with age: naive (CD45RA+CCR7+) cytotoxic T cells, exhausted (CD28-CD45RA-) cytotoxic T cells, and plasma B cells. Based on the weights used in the average, one can distinguish two types of measures of *EEAA:* i) the static measure *EEAA.static* defined with respect to fixed weights (defined in the largest cohort), and ii) the dynamic measure *EEAA.dynamic,* which estimates the weights in each data set. Since *EEAA.dynamic* refits the relative weightings of blood cell counts in each data set, it only involves blood cell counts that correlate with chronological age in the respective data set. In practice, the dynamic measure is less attractive than the static measure since its definition changes from one data set to the next. By contrast, the definition of *EEAA.static* is fixed. In practice, the dynamic weighting has been found to change relatively little across data sets which explains why *EEAA.dynamic* is highly correlated with *EEAA.static* (r>0.90).

Both *EEAA.dynamic* and *EEAA.static* have a greater magnitude of association with all-cause mortality than AgeAccelHannum in univariate and multivariate Cox models. We denote the static measure *EEAA.static* simply as *EEAA* in the following. The measure of extrinsic epigenetic age acceleration *(EEAA)* aims to measure epigenetic ageing in immune-related components. *EEAA* is defined using the following three steps. First, the epigenetic age measure from Hannum et al (2013) [2] is calculated, which is weakly correlated with certain blood cell types [5]. Second, the contribution of blood cell types to the age estimate is increased by forming a weighted average of Hannum's estimate with 3 cell types that are known to change with age: naive (CD45RA+CCR7+) cytotoxic T cells, exhausted (CD28-CD45RA-) cytotoxic T cells, and plasma B cells using the approach of [30]. The weights used in the weighted average are determined by the correlation between the respective variable and chronological age [30]. Two types of averages are distinguished: the static and dynamic averages refer to the situation where the weights are fixed (according to values in the WHI) or estimated, respectively. The resulting static and dynamic estimates of age give rise to (static and dynamic) measures of epigenetic age. Third, the measures of extrinsic epigenetic age acceleration *(EEAA)* are defined as the residual variation resulting from a univariate model regressing epigenetic age on chronological age. To provide conservative estimates, we only used the static measure (EEAA. static) in our examples (Figures 2-8). The dynamic measures EEAA.dynamic led to more significant associations with mortality.

The resulting static *(EEAA.static)* and dynamic *(EEAA.dynamic)* measures of extrinsic age acceleration are highly correlated and are positively correlated with the estimated abundance of exhausted CD8+ T cells, plasma blast cells, and a negative correlated with naive CD8+ T cells. Blood cell counts were estimated based on DNA methylation data as described in "Estimating blood cell counts based on DNA methylation levels" in the Example section. By construction, the measures of *EEAA* track both age related changes in blood cell composition and intrinsic epigenetic changes. None of the four measures of epigenetic age acceleration are correlated with chronological age.

Blood cell proportions can be estimated, for example, using two different software tools. Houseman's estimation method [31], which is based on DNA methylation signatures from purified leukocyte samples, may be used to estimate the proportions of cytotoxic (CD8+) T cells, helper (CD4+) T, natural killer, B cells, and granulocytes. The software does not allow for the identification of the type of granulocytes in blood (neutrophil, eosinophil, or basophil) but it is noted that neutrophils tend to be the most abundant granulocyte (~60% of all blood cells compared with 0.5-2.5% for eosinophils and basophils). Another method (invented by Stefan Horvath) and described below, may be used to estimate the percentage of exhausted CD8+ T cells (defined as CD28-CD45RA-) and the number (count) of naive CD8+ T cells (defined as CD45RA+CCR7+).

One exemplary implementation for the invention is in the area of life insurance. Upon completing a medical exam, an insurer will look at the test results as well as other factors such as family health history and lifestyle choices to classify the applicant into a basic classification: 1) preferred plus/super preferred/preferred select/preferred elite, 2) preferred, 3) standard plus, 4) standard, 5) preferred smoker, 6) standard smoker, 7) table rate A, 8) table rate B, etc. Each of these categories has a distinct mortality risk and usually directly relates to the pricing of the insurance product. The basic classification is largely determined by well-established risk factors of mortality such as sex, smoking status, family history of death, prior history of disease (e.g. diabetes status, cancer), and a host of clinical biomarkers (blood pressure, body mass index, cholesterol, glucose levels, hemoglobin A1C). The profitability of a life insurance product directly depends on the accurate assessment of mortality risk because the costs of life insurance (to the insurance company) are directly proportional to the number of deaths in a given category. Thus, any improvement in assessing mortality risk and in improving the basic classification will directly translate into cost savings.

Furthermore, novel "biomarkers of aging", i.e. assessments that allow one to prognosticate mortality, are not only interesting to medical underwriters but also to gerontologists (aging researchers), epidemiologists, and medical professionals. It is useful to distinguish clinical biomarkers from molecular biomarkers of aging. Clinical biomarkers such as lipid levels, body mass index, blood pressures have a long and successful history in the life insurance industry. By contrast, molecular biomarkers of aging such as telomere length are rarely used. However, this is likely to change due to recent breakthroughs in the development of molecular biomarkers of aging. Since their inception in 2013, DNA methylation (DNAm) based biomarkers of aging are revolutionizing aging research. Recent scientific publications surrounding the prediction of mortality and frailty strongly suggest that these DNAm based biomarkers are likely to benefit the life insurance industry as well. While DNAm measurements will not replace traditional assessments, they provide a host of complementary information that can inform the medical underwriting process. DNAm based biomarkers can not only be used to directly predict/prognosticate mortality but can also be used to molecularly estimate complete blood counts and smoking status.

The invention describes an enhanced and predictor of mortality based on DNA methylation levels in blood or other sources of DNA. Individuals differ substantially in terms of their measures of age acceleration. By definition, the mean age acceleration across individuals of a given data set is zero. In one example, the estimated hazard ratio for *EEAA* (HR=1.040, Figure 2) suggests a value of *EEAA=10* is associated with a 48% increased hazard of death (1.48=1.040^10). About five percent of the participants of the Women's Health Initiative (WHI) exhibited an *EEAA* value larger than 10. About 20% of participants of the WHI exceeded an *EEAA* value of 5, which is associated with a 22% increase in the hazard of death (HR=1.22). Similar, calculations apply to individuals with negative age acceleration. For example, 20% of individuals have an *EEAA* value less than -5, which is associated with an 18% decrease in the hazard of death (HR=0.82=1.04^(-5)).

The invention is defined by the appended claims. Furthermore disclosed is a method of observing counts of at least one type of cell selected from naive cytotoxic T cells, exhausted cytotoxic T cells, and/or plasma B cells within a population of leukocytes obtained from an individual. Typically this method comprises obtaining the population of leukocytes from the individual and then observing a presence or absence of methyl moieties at a plurality of CpG markers that can be used to estimate the counts or proportions of naive cytotoxic T cells in the population of leukocytes obtained from the individual and/or counts/proportions of exhausted cytotoxic T cells in the population of leukocytes obtained from the individual, and/or counts/proportions of plasma B cells in the population of leukocytes obtained from the individual. The next step in this embodiment is forming a weighted average of methyl groups at the plurality of CpG markers in order to estimate the naive cytotoxic T cells, exhausted cytotoxic T cells, and/or plasma B cells in the individual.

Optionally the method further includes using the observed counts of at least one type of cell selected from naive cytotoxic T cells, exhausted cytotoxic T cells, and/or plasma B cells in the population of leukocytes obtained from an individual in combination with a DNAm age estimate to arrive at a biological age estimate. Typically the biological age of the individual is estimated using a weighted average of DNA methylation levels. In certain embodiments, the method comprises comparing the methylation status of the plurality of CpG markers observed in the population of leukocytes from the individual with the methylation status of the same markers from a age matched reference population so as to obtain a value or a range of values for cell counts of naive cytotoxic T cells, exhausted cytotoxic T cells, and/or plasma B cells and/or the DNA methylation age of the individual. These methods can further comprise obtaining the chronological age of the individual and comparing the chronological age with the estimated biological age or epigenetic age so as to observe a presence or absence of epigenetic age acceleration in the individual.

Typically the methods comprise observing a presence or absence of methyl groups at a plurality of CpG markers that can be used to estimate the count or proportion of naive cytotoxic T cells comprises observing at least 5 CpG methylation markers selected from: cg15867698, cg17478979, cg25289028, cg10909506, cg23001918, cg17820878, cg07107916, cg09025210, cg07899551, cg03370106, cg26485825, cg21097090, cg25130381, cg16662477, cg06952412, cg05392293, cg26720010, cg04683740, cg08945443, cg20386303, cg15535471, cg25020550, cg24376214, cg21593149, cg25639084, cg15989436, cg02033323, cg18346531, cg02989940, cg10274029, cg07955474, cg18442362, cg23876292, cg12966876, cg17850367, cg01372366, cg05913271, cg07094298 and cg10493055; and/or observing a presence or absence of methyl groups at a plurality of CpG markers that can be used to estimate the counts or proportions of exhausted cytotoxic T cells comprises observing at least 5 CpG methylation markers selected from: cg07094298, cg04683740, cg26655856, cg14518178, cg01372366, cg09197075, cg03132824, cg00147638, cg25020550, cg23731272, cg22513455, cg00495443, cg08688907, cg21912203, cg10688297, cg10909506, cg26091609, cg00073460, cg08454507, cg20723792, cg14969094, cg01124420, cg06445016, cg05217983, cg03467087, cg02988775, cg01345395, cg16549957, cg21593149, cg00871371, cg11685391, cg23001918, cg26485825, cg08482359 and cg13608166; and/or observing a presence or absence of methyl groups at a plurality of CpG markers that can be used to estimate the counts or proportions of plasma B cells comprises observing at least 5 CpG methylation markers selected from cg24735235, cg01372366, cg25521400, cg01124420, cg13553498, cg26164712, cg20244489, cg08945443, cg13608166 and cg06245711. Optionally at least 10 CpG methylation markers (or all of these markers) are observed.

Typically the methods comprise observing the presence or absence of methyl groups at a plurality of CpG markers by treating genomic DNA from the population of leukocytes with bisulfite to transform unmethylated cytosines of CpG dinucleotides in the genomic DNA to uracil. Certain embodiments of the invention include compositions comprising a plurality of polynucleotide sequences that are derived from genomic DNA (e.g. 5, 10, 15, 20 or more CpG sites identified as providing information on naive cytotoxic T cells, exhausted cytotoxic T cells, and/or plasma B cells that are identified in the paragraph immediately above) but which are not naturally occurring because the unmethylated cytosines of CpG dinucleotides in the genomic DNA have been converted to uracil. Optionally this genomic DNA is obtained from a population of leukocytes is obtained from blood or saliva of the individual.

Furthermore disclosed is a method for observing epigenetic age acceleration in an individual. Typically this method comprises observing methylation of a set of methylation markers in genomic DNA obtained from white blood cells from an individual; using the methylation patterns observed to estimate counts of naive cytotoxic T cells, exhausted cytotoxic T cells, and plasma B cells, and the DNA methylation age in the individual; and then using the estimated cell counts and DNA methylation age obtained to estimate the biological age of the individual; and then comparing this estimated biological age to the true chronological age of the individual so as to observe epigenetic age acceleration in the individual. Typically in these methods, the biological age of the individual is estimated using weighted averages of DNA methylation levels for the estimated cell counts of naive cytotoxic T cells, exhausted cytotoxic T cells, plasma B cells, and DNA methylation age in the individual. In certain embodiments, the method comprises comparing the methylation observed in the set of 5, 10, 15 or more methylation markers with the methylation status of the same markers from a correlated reference population of cells so as to obtain a value or a range of values for cell counts of naive cytotoxic T cells, exhausted cytotoxic T cells, and/or plasma B cells and/or the DNA methylation age of the individual. Typically, but not necessarily, observing the presence or absence of methyl groups at a plurality of CpG markers comprises treatment of genomic DNA from the population of leukocytes with bisulfite to transform unmethylated cytosines of CpG dinucleotides in the genomic DNA to uracil. Optionally the population of leukocytes is obtained from saliva of the individual.

The disclosure provides for methods for estimating the biological age of an individual based on DNA methylation measures comprising: (a) obtaining a biological sample of the individual; (b) determining the methylation status of one or more markers or sites in the genomic DNA that lend themselves for estimating blood cell counts and DNA methylation age; (c) and estimating the biological age as a weighted average of the blood cell count estimates and the DNA methylation age; and optionally (d) contrasting the biological age estimate with the expected biological age of a reference population using a statistical method such as multivariate regression method, linear regression analysis, tabular method, or graphical method. In certain embodiments of the invention, the method comprises use of a statistical method to compare the methylation status of a set of epigenetic marker(s) of the individual with the methylation status of the same markers from an age matched reference population. Examples of suitable statistical methods include but are not limited to multivariate regression method, linear regression analysis, tabular method or graphical method, variance analysis, entropy statistics, and/or Shannon entropy. In a preferred embodiment, the statistical method comprises a multivariate regression algorithm or linear regression algorithm.

Furthermore described is a method for determining the epigenetic age acceleration/aging rate of an individual. The method comprises measuring a methylation level of a set of methylation markers in genomic DNA of an individual and determining the biological age of the individual based on the measured methylation levels. The biological age of the individual is typically determined based on a weighted average of the DNA methylation levels that is useful to observe counts of at least one of a naive cytotoxic T cells, exhausted cytotoxic T cells, plasma B cells, and the DNA methylation age in the individual. The determined biological age is then compared to a chronological age of the individual to determine an epigenetic age acceleration of the individual. Instances wherein the biological age is greater than expected based on the chronological age of the individual is an indication of an increased risk of all-cause mortality.

As noted above, embodiments of the present disclosure observe methylated DNA in naive cytotoxic T cells, exhausted cytotoxic T cells, and plasma B cells in the individual. Naive cytotoxic T cells (CD45RA+CCR7+), exhausted cytotoxic T cells (CD28-CD45RA-) and are known in the art, see, e.g. Tomiyama et al., Eur J Immunol. 2004 Apr;34(4):999-1010; Albrect et al., Cancer Immunol Immunother. 2011 Feb;60(2):235-48. doi: 10.1007/s00262-010-0936-8. Epub 2010 Nov 3; and Matsuki et al. Biochem Biophys Res Commun. 2013 Sep 6;438(4):778-83. doi: 10.1016/j.bbrc.2013.05.120. Epub 2013 Jun 6; Lu et al., Oncotarget. 2016 May 17;7(20):28783-95. doi: 10.18632/oncotarget.8939; and Libri et la., Immunology. 2011 Mar;132(3):326-39. doi: 10.1111/j.1365-2567.2010.03386.x. Epub 2011 Jan 7. Plasma B develop from activated B cells and secrete large numbers of antibodies. A plasma cell is a fully differentiated, mature lymphocyte in the B cell lineage.

Furthermore, the biological age of the individual is based on weighted averages of the DNA methylation levels useful to observe counts of naive cytotoxic T cells, exhausted cytotoxic T cells, and plasma B cells, and the DNA methylatoin age in the individual. The invention can also be practiced without estimating plasma B cells. Thus, in other embodiments, the biological age of the individual is based on a weighted average of the DNA methylation levels useful to observe cells count of naive cytotoxic T cells, exhausted cytotoxic T cells, and the DNA methylation age in the individual. The biological age comprises methylation pattern based weights for the naive cytotoxic T cells, exhausted cytotoxic T cells, and DNA methylation age, and/or plasma B cells determined by the strength of the correlation between the respective naive cytotoxic T cells, exhausted cytotoxic T cells, and/or plasma B cells and the chronological age of the individual. The epigenetic age acceleration is positively correlated with the cell count of the exhausted cytotoxic T cells and plasma B cells and is negatively correlated with the cell count of the naive cytotoxic T cells. In typical embodiments, the biological age of the individual is determined by (a) obtaining a linear combination of the methylation marker levels, and (b) applying a transformation to the linear combination to determine the biological age of the individual.

In another embodiment of the disclosure, a method for observing the health of an individual is provided. The method comprises collecting a tissue sample from an individual and extracting genomic DNA from the collected tissue sample. A methylation level or status of a methylation marker on the genomic DNA is measured and the biological age of the individual is determined with a statistical prediction algorithm. The statistical prediction algorithm is applied to the measured methylation level or status to determine the biological age of the individual. A biological age of the individual is determined based on cell counts of naive cytotoxic T cells, exhausted cytotoxic T cells, and the DNA methylation age in the individual. The determined biological age is then compared to a chronological age of the individual. Instances where the biological age is greater than expected based on the chronological age of the individual is an indication of a higher mortality risk of the individual compared to other people of the same chronological age. In certain embodiments, determination of the biological age of the individual is further based on a cell count of plasma B cells in the individual.

Furthermore a tangible computer-readable medium is provided comprising computer-readable code that, when executed by a computer, causes the computer to perform operations comprising receiving information corresponding to methylation levels of a set of methylation markers in a biological sample. The computer-readable code further causes the computer to determine an epigenetic age by applying a statistical prediction algorithm to the set of methylation markers, determine the biological age based on a weighted average of the DNA methylation levels useful to observe cell counts of naive cytotoxic T cells, exhausted cytotoxic T cells, and DNA methylation age, and compare the determined biological age to a chronological age of the individual a the time of sample collection. In certain embodiments, determination of the biological age of the individual is further based on a cell count of plasma B cells in the individual. In certain instances, the step of receiving information comprises receiving from a tangible data storage device information corresponding to the methylation levels of the set of methylation markers in the biological sample. The tangible computer-readable medium may further comprise computer-readable code that, when executed by a computer, causes the computer to send information corresponding to the methylation levels of the set of methylation markers in the biological sample to a tangible data storage device.

As noted above, the invention describes a biological age estimate. To arrive at this estimate, one needs 4 input variables including DNAm age (e.g. one based on a Hannum type methodology, see, e.g. U.S. Patent Publication 20150259742). Typically, the biological age estimate (sometimes denoted BioAge4) is the weighted average of 4 quantities (hence the number 4 in its name). These quantities are i) DNAm age based on a Hannum type methodology or another procedure, ii) naïve CD8+ T cells, iii) exhausted CD8T cells, and iv) plasma blasts. The plasma blast estimate is optional, i.e. one could estimate biological age based on three input variables.

Certain sets of CpGs lend themselves for estimating naive CD8+, exhausted CD8T cells, and plasma blasts. The graphic in Figure 18 illustrates a working embodiment of this, i.e. reduces it to practice. Such embodiments of the invention focus on estimating biological age (e.g. by supplementing a Hannum type estimate of epigenetic age with blood cell counts) and not about estimating blood cell counts using DNA methylation levels. Figure 18 shows flow cytometric measures of blood cell counts versus estimated blood cell counts based on DNA methylation levels. A) The predicted/estimated count of naive CD8+ T cells (x-axis) is highly correlated with the observed count based on flow cytometric measures. B) Analogous graph for the percentage of exhausted CD8+ T cells defined as CD28 negative, CD45RA negative markers. C) Analogous plot for percentage of plasma blasts. The flow cytometric data and DNA methylation data were assessed in 96 individuals between 30 and 70 years of age who were not used in the development of the method, i.e. they represent an unbiased validation set.

As noted above, embodiments of the present invention relate to methods for estimating the biological age of an individual human tissue or cell type sample based on measuring DNA Cytosine-phosphate-Guanine (CpG) methylation markers that are attached to DNA. In a general embodiment of the invention, a method is disclosed comprising a first step of choosing a source of DNA such as specific biological cells (e.g. T cells in blood) or tissue sample (e.g. blood) or fluid (e.g. saliva). In a second step, genomic DNA is extracted from the collected source of DNA of the individual for whom a biological age estimate is desired. In a third step, the methylation levels of the methylation markers near the specific clock CpGs are measured. In a fourth step, a statistical prediction algorithm is applied to the methylation levels to predict the age. One basic approach is to form a weighted average of the CpGs, which is then transformed to DNA methylation (DNAm) age using a calibration function. As used herein, "weighted average" is a linear combination calculated by giving values in a data set more influence according to some attribute of the data. It is a number in which each quantity included in the linear combination is assigned a weight (or coefficient), and these weightings determine the relative importance of each quantity in the linear combination.

DNA methylation of the methylation markers (or markers close to them) can be measured using various approaches, which range from commercial array platforms (e.g. from IlluminaTM) to sequencing approaches of individual genes. This includes standard lab techniques or array platforms. A variety of methods for detecting methylation status or patterns have been described in, for example U.S. Pat. Nos. 6,214,556, 5,786,146, 6,017,704, 6,265,171, 6,200,756, 6,251,594, 5,912,147, 6,331,393, 6,605,432, and 6,300,071 and US Patent Application Publication Nos. 20030148327, 20030148326, 20030143606, 20030082609 and 20050009059.

Other array-based methods of methylation analysis are disclosed in U.S. patent application Ser. No. 11/058,566. For a review of some methylation detection methods, see, Oakeley, E. J., Pharmacology & Therapeutics 84:389-400 (1999). Available methods include, but are not limited to: reverse-phase HPLC, thin-layer chromatography, SssI methyltransferases with incorporation of labeled methyl groups, the chloracetaldehyde reaction, differentially sensitive restriction enzymes, hydrazine or permanganate treatment (m5C is cleaved by permanganate treatment but not by hydrazine treatment), sodium bisulfite, combined bisulphate-restriction analysis, and methylation sensitive single nucleotide primer extension.

The methylation levels of a subset of the DNA methylation markers disclosed herein are assayed (e.g. using an IlluminaTM DNA methylation array, or using a PCR protocol involving relevant primers). To quantify the methylation level, one can follow the standard protocol described by IlluminaTM to calculate the beta value of methylation, which equals the fraction of methylated cytosines in that location. The invention can also be applied to any other approach for quantifying DNA methylation at locations near the genes as disclosed herein. DNA methylation can be quantified using many currently available assays which include, for example:
a) Molecular break light assay for DNA adenine methyltransferase activity is an assay that is based on the specificity of the restriction enzyme DpnI for fully methylated (adenine methylation) GATC sites in an oligonucleotide labeled with a fluorophore and quencher. The adenine methyltransferase methylates the oligonucleotide making it a substrate for DpnI. Cutting of the oligonucleotide by DpnI gives rise to a fluorescence increase.
b) Methylation-Specific Polymerase Chain Reaction (PCR) is based on a chemical reaction of sodium bisulfite with DNA that converts unmethylated cytosines of CpG dinucleotides to uracil or UpG, followed by traditional PCR. However, methylated cytosines will not be converted in this process, and thus primers are designed to overlap the CpG site of interest, which allows one to determine methylation status as methylated or unmethylated. The beta value can be calculated as the proportion of methylation.
c) Whole genome bisulfite sequencing, also known as BS-Seq, is a genome-wide analysis of DNA methylation. It is based on the sodium bisulfite conversion of genomic DNA, which is then sequencing on a Next-Generation Sequencing (NGS) platform. The sequences obtained are then re-aligned to the reference genome to determine methylation states of CpG dinucleotides based on mismatches resulting from the conversion of unmethylated cytosines into uracil.
d) The Hpall tiny fragment Enrichment by Ligation-mediated PCR (HELP) assay is based on restriction enzymes' differential ability to recognize and cleave methylated and unmethylated CpG DNA sites.
e) Methyl Sensitive Southern Blotting is similar to the HELP assay but uses Southern blotting techniques to probe gene-specific differences in methylation using restriction digests. This technique is used to evaluate local methylation near the binding site for the probe.
f) ChIP-on-chip assay is based on the ability of commercially prepared antibodies to bind to DNA methylation-associated proteins like MeCP2.
g) Restriction landmark genomic scanning is a complicated and now rarely-used assay is based upon restriction enzymes' differential recognition of methylated and unmethylated CpG sites. This assay is similar in concept to the HELP assay.
h) Methylated DNA immunoprecipitation (MeDIP) is analogous to chromatin immunoprecipitation. Immunoprecipitation is used to isolate methylated DNA fragments for input into DNA detection methods such as DNA microarrays (MeDIP-chip) or DNA sequencing (MeDIP-seq).
i) Pyrosequencing of bisulfite treated DNA is a sequencing of an amplicon made by a normal forward primer but a biotinylated reverse primer to PCR the gene of choice. The Pyrosequencer then analyses the sample by denaturing the DNA and adding one nucleotide at a time to the mix according to a sequence given by the user. If there is a mismatch, it is recorded and the percentage of DNA for which the mismatch is present is noted. This gives the user a percentage methylation per CpG island.

In certain embodiments of the invention, the genomic DNA is hybridized to a complimentary sequence (e.g. a synthetic polynucleotide sequence) that is coupled to a matrix (e.g. one disposed within a microarray). Optionally, the genomic DNA is transformed from its natural state via amplification by a polymerase chain reaction process. For example, prior to or concurrent with hybridization to an array, the sample may be amplified by a variety of mechanisms, some of which may employ PCR. See, for example, PCR Technology: Principles and Applications for DNA Amplification (Ed. H. A. Erlich, Freeman Press, NY, N.Y., 1992); PCR Protocols: A Guide to Methods and Applications (Eds. Innis, et al., Academic Press, San Diego, Calif., 1990); Mattila et al., Nucleic Acids Res. 19, 4967 (1991); Eckert et al., PCR Methods and Applications 1, 17 (1991); PCR (Eds. McPherson et al., IRL Press, Oxford); and U.S. Pat. Nos. 4,683,202, 4,683,195, 4,800,159, 4,965,188, and 5,333,675. The sample may be amplified on the array. See, for example, U.S. Pat. No. 6,300,070.

In addition to using art accepted modeling techniques (e.g. regression analyses), embodiments of the invention can include a variety of art accepted technical processes. For example, in certain embodiments of the invention, a bisulfite conversion process is performed so that cytosine residues in the genomic DNA are transformed to uracil, while 5-methylcytosine residues in the genomic DNA are not transformed to uracil. Kits for DNA bisulfite modification are commercially available from, for example, MethylEasyTM (Human Genetic SignaturesTM) and CpGenomeTM Modification Kit (ChemiconTM). See also, WO04096825A1, which describes bisulfite modification methods and Olek et al. Nuc. Acids Res. 24:5064-6 (1994), which discloses methods of performing bisulfite treatment and subsequent amplification. Bisulfite treatment allows the methylation status of cytosines to be detected by a variety of methods. For example, any method that may be used to detect a SNP may be used, for examples, see Syvanen, Nature Rev. Gen. 2:930-942 (2001). Methods such as single base extension (SBE) may be used or hybridization of sequence specific probes similar to allele specific hybridization methods. In another aspect the Molecular Inversion Probe (MIP) assay may be used.

### EXAMPLES

### EXAMPLE 1: DNA Methylation-Based Measures of Biological Age: Meta-Analysis Predicting Time to Death

Aspects of this disclosure are published in a technical journal as Chen et al., 2016 Sep 28;8(9): 1844-1865. doi: 10.18632/aging.101020].

Estimates of biological age based on DNA methylation patterns, often referred to as "epigenetic age" or "DNAm age," have been shown to be robust biomarkers of age in humans. We previously demonstrated that independent of chronological age, epigenetic age assessed in blood predicted all-cause mortality in four human cohorts. Here, we expanded our original observation to 13 different cohorts for a total sample size of 13,089 individuals, including three racial/ethnic groups. In addition, we examined whether incorporating information on blood cell composition into the epigenetic age metrics improves their predictive power for mortality. All considered measures of epigenetic age acceleration were predictive of mortality (p≤8.2×10⁻⁹), independent of chronological age, even after adjusting for additional risk factors (p<5.4×10⁻⁴), and within the racial/ethnic groups that we examined (non-Hispanic whites, Hispanics, African Americans). Epigenetic age estimates that incorporated information on blood cell composition led to the smallest p-values for time to death (p=7.5×10⁻⁴³). Overall, this study a) strengthens the evidence that epigenetic age predicts all-cause mortality above and beyond chronological age and traditional risk factors, and b) demonstrates that epigenetic age estimates that incorporate information on blood cell counts lead to highly significant associations with all-cause mortality.

DNA methylation-based biomarkers, often referred to as "epigenetic age" are robust estimators of chronological age of an individual [1-4]. For example, a measure of epigenetic age based on levels of methylation in 353 CpG dinucleotide markers (cytosine linked to guanine by a phosphate group) allow the estimation of the age of an individual. This estimate is consistent across most types of biological specimens, including whole blood, brain, breast, kidney, liver, lung, and saliva and cell types, including CD4+ T cells, monocytes, B cells, glial cells, and neurons [3].

Recent studies suggested that epigenetic age is associated with age-related health outcomes above and beyond chronological age. For example, we and others have shown that individuals whose epigenetic age was greater than their chronological age (i.e., individuals exhibiting epigenetic "age acceleration") were at an increased risk for death from all causes, even after accounting for known risk factors [5-7]. Further, we recently showed that the offspring of semi-supercentenarians (individuals who reached an age of 105-109 years) have a lower epigenetic age than age-matched controls [8]. Based on these findings, it has been hypothesized that epigenetic age captures some aspect of biological age and the resulting susceptibility to disease and multiple health outcomes. A first step in testing this hypothesis is to test whether epigenetic age predicts longevity in multiple populations and across ethnic groups.

In many studies epigenetic age is estimated from DNA derived from blood samples. It is well known that blood cell composition changes with age and some of these changes might be independent predictors of mortality [9-12]. Thus, it is of interest to understand whether considering information on blood cell composition in measures of epigenetic age improves their predictive power for mortality.

Here, we evaluated the ability to predict time to death for blood-based epigenetic age measures, both published and novel measures that incorporate information on cell composition. Due to the well documented age-related changes in blood cell composition, we distinguished epigenetic measures of age that were independent of changes in blood cell composition (cell-intrinsic epigenetic age), and measures that incorporated age-related changes in blood cell composition in its age estimation ("extrinsic" epigenetic age). By increasing the number of independent cohort studies, we more than doubled the number of mortality events available for analysis, which allowed for detailed subgroup analyses, such as the examination of racial/ethnic differences.

### Results

### Cohort studies

Our meta-analysis included 13 population-based cohorts. An overview of the cohorts is provided in **Figure 9****.** Our study involved 3 racial/ethnic groups: non-Hispanic whites (n=9,215), Hispanics (n=431), and Blacks (n=3,443).

### Epigenetic age estimation

We used two methods for estimating the epigenetic age of each blood sample **(****Figure 10****).** First, we used the approach by Horvath (2013) based on 353 CpGs, as described in [3] and **Methods.** Second, we used the approach by Hannum et al. (2013) based on 71 CpGs [2]. Both epigenetic age estimates were correlated with chronological age at the time of blood draw **(****Figure 9****)** with biweight midcorrelation coefficients ranging from 0.65 to 0.89 (birth cohorts were excluded from this analysis). The Horvath and Hannum estimates were also highly correlated with each other (r=0.76) even though the underlying sets of CpGs share only 6 CpGs in common.

### (Figure 13).

### Estimated blood cell counts that relate to chronological age

We estimated the abundance of ten blood cell types based on observed DNA methylation patterns **(Methods)** -exhausted/senescent CD8+ T cells (CD8+CD28-CD45RA-), CD8+ naive, CD8+ total, CD4+ naive, CD4+ total, natural killer cells, B cells, monocytes, granulocytes, and plasma B cells. To study age-related changes in blood cell composition, we correlated these estimated blood cell counts with chronological age in all of the cohort studies **(****Figure 14****).** Our results are congruent with findings from flow cytometric studies that demonstrate that the abundance of naive CD8+ T cells decreases with age (reflecting thymic involution), whereas exhausted/senescent CD8+ T cells increase with age [9-12].

### Measures of epigenetic age acceleration

Despite high correlations, epigenetic age can deviate substantially from chronological age at the individual level. The difference between epigenetic age and chronological age can be used to define "delta age" but the resulting measure exhibits a negative correlation with chronological age. By contrast, all of our measures of epigenetic age acceleration are defined such that they uncorrelated with chronological age. One such measure (denoted as *AgeAccel*) is defined as the residual that results from regressing epigenetic age on chronological age. Thus, a positive value of *AgeAccel* indicates that the epigenetic age is higher than expected, based on chronological age. These Horvath and Hannum based measures of age acceleration are denoted by *AgeAccel_{Horvath}* and *AgeAccel_{Hannum}*, respectively. For the sake of brevity and consistency with other publications from our group, we abbreviate *AgeAccel_{Horvath}* as *AgeAccel.*

*AgeAccel_{Hannum}* and to a lesser extent *AgeAccel* were previously shown to correlate with blood cell counts [5]. Thus, we distinguished two broad categories of measures of epigenetic age acceleration when dealing with DNA methylation from blood or peripheral blood mononuclear cells (PBMCs): intrinsic and extrinsic epigenetic measures, which are independent of, or enhanced by blood cell count information, respectively **(**Figures 15A-B). We define *intrinsic* epigenetic age acceleration *(IEAA)* as the residual resulting from regressing epigenetic age on chronological age and measures of blood cell counts **(Methods).** By definition, *IEAA* is not correlated with chronological age and is weakly correlated with estimated measures of blood cell counts **(****Figure 16****).** *IEAA* is meant to capture cell-intrinsic properties of the aging process that exhibit some preservation across various cell types and organs. Compared to our other measures of age acceleration, *IEAA,* adapted from the Horvath measure of epigenetic age, exhibited significant correlations with epigenetic age acceleration in breast tissue (r=0.48, p=0.0011, **Figure 1B****)** and saliva (r=0.67, p=8.8×10⁻⁹, **Figure 1F****).** By contrast, an analogous measure of *IEAA* based on the Hannum measure showed much weaker correlations (r=0.073 in breast and r=0.41 in saliva **Figure 1D**, 1H). For this reason, we focused on the Horvath measure of *IEAA.*

The age-related changes to blood cell composition **(****Figure 16****)** can be leveraged to capture aspects of immunosenescence. Using these measures, we derived a novel extrinsic epigenetic age acceleration *(EEAA)* measure by up-weighting the blood cell count contributions of *AgeAccel_{Hannum}* **(****Figure 16****).**

Descriptive statistics (minimum, maximum, median) that describe the distribution of the different measures of epigenetic age acceleration can be found in Figures 15A-B. By definition, all measures of epigenetic age acceleration have a mean value of zero. A summary of age acceleration definitions can be found in **Figure 10****.**

### Cox regression models of all-cause mortality

We used Cox regression models to assess the predictive value of our measures of epigenetic age acceleration for all-cause mortality. All of our Cox models were adjusted for the age at baseline (blood draw). Additional multivariate models further adjusted for covariates assessed at baseline (chronological age, body mass index, educational level, alcohol intake, smoking pack-years, prior history of diabetes, prior history of cancer, hypertension status, self-reported recreational physical activity).

Our novel measure of extrinsic age acceleration *EEAA* led to smaller p-values for the associations with all-cause mortality than the original measure *AgeAccel_{Hannum}* in univariate Cox models (p*_{EEAA}*=7. 5×10⁻⁴³, p*_{AgeAccelHannum}*=1.4×10⁻³⁴, **Figure 5****)** and in multivariate Cox models (p*_{EEAA}*=3.4×10⁻¹⁹, p*_{AgeAccelHannum}*=6×10⁻¹⁵, **Figure 6****).** Further, when both *EEAA* and *AgeAccel_{Hannum}* were included in the same Cox model, only *EEAA* remained significant in the WHI data and FHS univariate models. Due to the lack of evidence that *AgeAccel_{Hannum}* had greater predictive power for time to death than *EEAA,* we removed *AgeAccel_{Hannum}* from the subsequent analyses comparing extrinsic and intrinsic measures of age acceleration.

All considered measures of epigenetic age acceleration were predictive of time to death in univariate Cox models (*p_{AgeAccel}*=1.9×10^{*-*11}, p*_{IEAA}*=8.2×10⁻⁹, p*_{EEAA}*=7.5×10⁻⁴³, **Figure 2****)** and multivariate Cox models adjusting for risk factors and pre-existing disease status (p*_{AgeAccel}*=5.4×10⁻⁵, p*_{IEAA}*=5.0×10⁻⁴, p*_{EEAA}*=3.4×10⁻¹⁹, **Figure 3****).**

### Interpreting effect sizes and variance of epigenetic age acceleration

Individuals differed substantially in terms of their measures of epigenetic age acceleration, e.g. *EEAA* ranged from -28 to 28 years in the WHI (standard deviation =6.4 years, Figures 15A-B).

About five percent of the participants of the WHI exhibited an *EEAA* value larger than 10, which is associated with a 48% increased hazard of death (according to **Figure 2****,** the HR of *EEAA* is 1.040 which results in 1.48=(1.040)¹⁰). Negative values of age acceleration were associated with a lower hazard of mortality. For example, 20% of individuals had an *EEAA* value less than -5, which is associated with an 18% decrease in the hazard of death (HR=0.82=1.04⁻⁵).

### Subgroup analysis

With few exceptions, we found that the associations between *EEAA* and time to death remained highly significant in subgroups stratified by race, sex, follow-up duration, body mass index, smoking status, physical activity **(**Figures 11A-B) and in subgroups stratified by prevalent disease at baseline such as cancer, coronary artery disease, hypertension and type 2 diabetes **(****Figure 12****).** Only one subgroup led to an insignificant finding (p>0.05) in our univariate model analysis: namely individuals with less than 5 years of follow up **(**Figures 11A-B). For multivariate models, we failed to observe significant associations for the following subgroups: i) less than 5 years of follow up, ii) between 5 and 10 years of follow up, iii) current smokers, iv) obese individuals, v) Hispanics, vi) individuals with cancer, and vii) individuals with coronary artery disease. The insignificant results in multivariate models in cancer patients or CAD patients might reflect the relatively low sample sizes or that epigenetic age acceleration is dwarfed by other predictors of mortality in individuals with severe diseases. Hazard ratio estimates remained highly consistent across all subgroups examined.

We did not observe significant differences in the estimated hazard ratios across any subgroup **(**Figures 11A-B, 12). Specifically, racial/ethnic differences in HR were not observed (interaction p=0.62 in age-adjustment models and p=0.14 in full models). Overall, these subgroup analysis results confirm that epigenetic age acceleration is an independent predictor of earlier mortality even after adjusting for possible confounders and within major subgroups of the population.

### Hazard ratio of death versus follow up time and median age

The large number of cohorts allowed us to relate cohort characteristics (such as median age or median follow up time) to strength of association with mortality. We did not find a statistically significant relationship between the hazard ratio of death for the median age of the cohort or the follow up time **(****Figure 4****).**

### Robustness analysis

To assess the robustness of our findings, we also carried out a leave-one-out analysis by re-running the meta-analysis after removing data from individual cohorts. The resulting p-values are highly robust with respect to removing a single data set from the analysis **(****Figure 17****).** In our study, we used a fixed effects meta-analysis method for the sake of consistency with previous analyses [13]. However, our results remain qualitatively the same after using a random effects meta-analysis method

### (Figure 8).

### Methods

### Measures of epigenetic age

We used an epigenetic biomarker of age based on 353 CpG markers as one measure of epigenetic age because: a) it is an accurate measurement of age across multiple tissues [3]; b) we previously showed that it is predictive of all-cause mortality [5]; c) it correlated with measures of cognitive/physical fitness and neuropathology in the elderly [21, 22]; and d) it was associated with conditions that may represent accelerated aging, including Down's syndrome [23], Parkinson's disease [24], obesity [25], and HIV infection [26], and aging in centenarians [27]. This epigenetic age estimator not only lends itself to measuring aging effects in elderly individuals; but also applies to prenatal brain samples [28] and blood samples from minors [29]. Epigenetic age is defined as the predicted value of age based on the DNA methylation levels of 353 CpGs. Mathematical details and software tutorials for estimating epigenetic age can be found in the additional files of [3]. All of the described epigenetic measures of aging and age acceleration are implemented in our freely available software (https://dnamage.genetics.ucla.edu) [3].

### DNA methylation age estimate by Hannum et al (2013)

We also used an alternative measure of epigenetic age developed by Hannum et al (2013) [2]. The resulting age estimate is based on the 71 CpGs and coefficient values from the third Figures 15A-B [2]. The authors developed this age prediction method by using an elastic net regression model for predicting chronological age based on DNA methylation levels from whole blood.

### Measures of epigenetic age acceleration

**Figure 10** provides an overview of our measures of epigenetic age acceleration. The universal measure of age acceleration *(AgeAccel),* which is valid for a wide range of tissue types, is defined as the residual resulting from a linear regression model that regresses the Horvath estimate of epigenetic age on chronological age. Thus, a positive value for *AgeAccel* indicates that the observed epigenetic age is higher than that predicted, based on chronological age. *AgeAccel* has a relatively weak correlation with blood cell counts [26], but it still relates to estimated blood cell counts, as seen in **Figure 16****.**

To estimate "pure" epigenetic aging effects that are not influenced by differences in blood cell counts ("intrinsic" epigenetic age acceleration, *IEAA*), we obtained the residual resulting from a multivariate regression model of epigenetic age on chronological age and various blood immune cell counts (naive CD8+ T cells, exhausted CD8+ T cells, plasma B cells, CD4+ T cells, natural killer cells, monocytes, and granulocytes) imputed from methylation data.

Extrinsic epigenetic age acceleration measures capture both cell intrinsic methylation changes and extracellular changes in blood cell composition. Our measure of *EEAA* is defined using the following three steps. First, we calculated the epigenetic age measure from Hannum et al [2], which already correlated with certain blood cell types [5]. Second, we increased the contribution of immune blood cell types to the age estimate by forming a weighted average of Hannum's estimate with 3 cell types that are known to change with age: naive (CD45RA+CCR7+) cytotoxic T cells, exhausted (CD28-CD45RA-) cytotoxic T cells, and plasma B cells using the Klemera Doubal approach [30]. The weights used in the weighted average are determined by the correlation between the respective variable and chronological age [30]. The weights were chosen on the basis of the WHI data. Thus, the same (static) weights were used for all data sets. *EEAA was* defined as the residual variation resulting from a univariate model regressing the resulting age estimate on chronological age. By construction, *EEAA* is positively correlated with the estimated abundance of exhausted CD8+ T cells, plasma B cells, and a negative correlated with naive CD8+ T cells. Blood cell counts were estimated based on DNA methylation data as described in the next section. By construction, the measures of *EEAA* track both age related changes in blood cell composition and intrinsic epigenetic changes. None of our four measures of epigenetic age acceleration are correlated with chronological age.

### Estimating blood cell counts based on DNA methylation levels

We estimate blood cell proportions using two different software tools. Houseman's estimation method [31], which is based on DNA methylation signatures from purified leukocyte samples, was used to estimate the proportions of cytotoxic (CD8+) T cells, helper (CD4+) T, natural killer, B cells, and granulocytes. The software does not allow us to identify the type of granulocytes in blood (neutrophil, eosinophil, or basophil) but we note that neutrophils tend to be the most abundant granulocyte (~60% of all blood cells compared with 0.5-2.5% for eosinophils and basophils). The advanced analysis option of our epigenetic age calculator software [3] was used to estimate the percentage of exhausted CD8+ T cells (defined as CD28-CD45RA-) and the number (count) of naive CD8+ T cells (defined as CD45RA+CCR7+).

### Cox regression models and meta-analysis

Here, we used Cox models for analyzing the censored survival time data (from the age at blood draw until age at death or last follow-up). We regressed the censored survival times on covariates using Cox regression models implemented in the R function *coxph* in the *survival* package. The resulting coefficient values (interpreted as log hazard ratios) and standard errors were combined using the R software package *metafor* [32]. The meta-analysis was carried out with the R command *rma* (with arguments *method="FE"* to get fixed effects estimates). The forest plots were created using the R function *forest* (with argument *atransf=exp* to exponentiate the estimate of the log hazard ratios).

### Sample exclusions

In addition to cohort-specific quality checks, we further excluded individuals who had ever been diagnosed with leukemia (ICD-9: 203-208), reported receiving chemotherapy, and whose methylation beta value distributions deviated substantially from a gold standard (according to the quality statistic *corSampleVSgoldstandard<0.80* from the online age calculator [33-35]).

### DNA methylation quantification

In brief, bisulfite conversion using the Zymo EZ DNA Methylation Kit (Zymo Research, Orange, CA, USA) as well as subsequent hybridization of the HumanMethylation450k Bead Chip (Illumina, San Diego, CA), and scanning (iScan, Illumina) were performed according to the manufacturers protocols by applying standard settings. DNA methylation levels ( values) were determined by calculating the ratio of intensities between methylated (signal A) and un- methylated (signal B) sites. Specifically, the value was calculated from the intensity of the methylated (M corresponding to signal A) and un-methylated (U corresponding to signal B) sites, as the ratio of fluorescent signals = Max(M,0)/[Max(M,0)+Max(U,0)+100]. Thus, values range from 0 (completely un-methylated) to 1 (completely methylated).

In one example, DNA was extracted from 514 whole blood samples in LBC 1921. Samples were extracted at MRC Technology, Western General Hospital, Edinburgh (LBC 1921) and the Wellcome Trust Clinical Research Facility (WTCRF). Methylation typing was performed at the WTCRF using the Illumina HumanMethylation450 array. Raw intensity data were background-corrected and methylation beta-values generated using the R mmfi package . Manual inspection of the array control probe signals was used to identify and remove low quality samples (e.g., samples with inadequate hybridization, bisulfite conversion, nucleotide extension, or staining signal). The Illumina-recommended threshold was used to eliminate samples with a low call rate (samples ·with <450,000 probes detected at P <0.01). Since the LBC samples had previously been genotyped using the Illumina 610-Quadvl genotyping platforn1, genotypes derived from the 65 SNP control probes on the methylation array using the *·watermelon* R package⁵ were compared to those obtained from the genotyping array to ensure sample integrity. Samples with a low match of genotypes with SNP control probes, which could indicate sample contamination or mix-up, were excluded (n = 9). Moreover, eight individuals whose predicted sex, based on XY probes, did not match reported sex were also excluded.

In another example, genomic DNA was extracted from peripheral blood leukocyte samples usmg the Gentra Puregene Blood Kit (Qiagen; Valencia, CA, USA) according to the manufacturer's instrnctions (\vwi.:v.qiagen.com). Bisulfite conversion of 1 ug genomic DNA was performed using the EZ-96 DNA Methylation Kit (Deep Well Format) (Zymo Research; Irvine, CA, USA) according to the manufacturer's instrnctions (www.zymoresearch.com). Bisulfite conversion efficiency was detem1ined by PCR amplification of the conve1ied DNA before proceeding with methylation analyses on the Illumina platform using Zymo Research's Universal Methylated Human DNA Standard and Control Primers. The Illumina Infinium HumanMethylation450K Beadchip array (HM450K) was used to measure DNA methylation (Illumina, Inc.; San Diego, CA, USA). Background subtraction was conducted with the GenomeStudio software using built-in negative control bead types on the array. Positive and negative controls and sample replicates were included on each 96-well plate assayed. After exclusion of controls, replicates and samples with integrity issues or failed bisulfite conversion, a total of 2841 study participants had HM450K data available for further QC analyses. We removed poor-quality samples with pass rate of <99%, that is, if the sample had at least 1% of CpG sites with detection P-value > 0.01 or missing, indicative of lower DNA quality or incomplete bisulfite conversion, and samples with a possible gender mismatch based on evaluation of selected CpG sites on the Y chromosome.

The data presented in this Example corroborates previous findings regarding the predictive power of DNA methylation-based biomarkers of age for mortality [5, 6, 14]. We further examined novel variants of these measures that are either independent of blood cell counts or are enhanced by changes in blood cell sub-populations. We showed that the latter approximates, and in some cases, out-performs previous epigenetic age measures when comes to predicting all-cause mortality. Furthermore, the associations between epigenetic age acceleration and mortality did not differ significantly across subgroups of race/ethnicity, sex, BMI, smoking status, physical activity status, or major chronic diseases. The consistency of the associations across multiple subgroups lends support to the notion that epigenetic age acceleration captures some aspect of biological aging over and above chronological age and other risk factors.

The development of suitable measures of biological age has been a key goal in the field of aging research [15]. Many biomarkers of age have been posited including epigenetic alterations of the DNA (e.g., DNA methylation), transcriptomics [16], telomere length, whole-body function such as gait speed (reviewed in [17]), and composite indices of clinical variables [18]. The current study does not aim to replace existing blood based biomarkers, but rather, we aimed to demonstrate that epigenetic age captures some aspect of biological age, as assessed through lifespan, above and beyond the effect of chronological age. And measures of epigenetic age acceleration are attractive because they are highly robust and because their measurement only involve DNA methylation data.

While actual flow cytometry data will always be preferable to imputed blood cell count data (based on DNA methylation data), the measures of age acceleration do not require the measurement of flow data. Rather, measures of intrinsic and extrinsic epigenetic age used blood cell count estimates resulting from DNA methylation data. The measure of extrinsic epigenetic age acceleration probably reflects aspects of immunosenescence because, by construction, they correlate with age-related changes in blood cell composition, such as T lymphocyte populations, which underlie much of the age-related decline in the protective immune response [9-12]. Thus, the high predictive significance of *EEAA* for all-cause mortality probably reflects the fact that it assesses multiple aspects of the biological age of the immune system. It has been known for decades that poor T cell functioning is predictive of mortality [19]. In contrast, the findings surrounding the predictive utility of intrinsic epigenetic age acceleration are biologically compelling and point to a new frontier in aging research.

Our study strongly suggests *IEAA* is reflective of an intrinsic epigenetic clock that is associated with mortality independent of chronological age and blood cell composition. *IEAA* probably captures a cell-type independent component of the aging process for the following reasons. First, *IEAA* is moderately preserved across different tissues and cell types collected from the same individual **(****Figure 1****).** Second, *IEAA* but not *EEAA* is predictive of lung cancer [20]. Third, only *IEAA* and *AgeAccel* relate to centenarian status [14].

Overall, our results inform the ongoing debate about whether epigenetic biomarkers of age capture an aspect of biological age. While epigenetic processes are unlikely to be the only mediators of chronological age on mortality-in fact, multiple risk factors have stronger effects on mortality-our results suggest that at least one of the mediating processes relates to the epigenetic age of blood tissue and that this process is independent of age-dependent changes in blood cell composition. Future studies will be useful for gaining a mechanistic understanding of this intrinsic epigenetic aging process.

### EXAMPLE 2: ILLUSTRATIVE METHODS AND MATERIALS USEFUL FOR MEASURING THE DNA METHYLATION AGE OF A TISSUE SAMPLE AND ESTIMATING DNA METHYLATION-BASED PREDICTORS OF MORTALITY

### Step 1: Obtain cells from blood, saliva, or other sources of DNA from an individual.

There are several options for obtaining leukocytes for use in the methods disclosed herein such as those discussed below.

Blood collected by venipuncture. Blood collected by venipuncture will result in a large amount of high quality DNA from a relevant tissue. The invention applies to DNA from whole blood, or peripheral blood mononuclear cells or even sorted blood cell types. Dried blood spots can be easily collected by a finger prick method. The resulting blood droplet can be put on a blood card, e.g. http://www.lipidx.com/dbs-kits/. Saliva. Unexpectedly, saliva also contains amounts of leukocytes is a condition that allows them to be used in methods of the invention.

### Step 2: Generate DNA methylation data

This step can be carried out by the lab that collects the tissue or DNA samples. An illustrative methodology is discussed below.

Step 2a: Extract the genomic DNA from the cells.

Step 2b: Measure cytosine DNA methylation levels.
Several approaches can be used for measuring DNA methylation including sequencing, bisulfite sequencing, arrays, pyrosequencing, liquid chromatography coupled with tandem mass spectrometry.

### Step 3: Estimate the DNA methylation age

Many different approaches could be used for estimating the age of an individual based on cytosine methylation levels, e.g. one can use the 71 CpGs from Hannum et al 2013. The Hannum estimator is based on forming a weighted average of the DNA methylation levels of 71 CpGs. See e.g. U.S. Patent Publication 20150259742 and Hannum et al., Molecular cell. 2013; 49(2):359-367.

The resulting age estimate can be denoted "epigenetic age" or "apparent methylomic aging rate" or "DNAmAge".

### Step 4: Estimate the abundance of 3 blood cell types.

One embodiment of the invention requires that one estimate the abundance of three blood cell types: plasma blasts (denoted PlasmaBlast) exhausted cytotoxic T cells (denoted CD8pCD28nCD45RAn) and naive cytotoxic T cells (denoted CD8.naive). While flow cytometric methods could be used to quantify the abundance of these blood cell types, one can also use DNA methylation data. To estimate the blood cell counts based on DNAm data, one measures the methylation levels of the respective sets of CpGs. Next one forms a weighted average of the methylation levels using the reported coefficient values and adds an intercept term.

### Step 5: Standardize the values of the four input variables DNAmAge, PlasmaBlast, CD8pCD28nCD45RAn, CD8.naive.

Each input variable can be standardized by subtracting a value (referred to as center) and dividing the resulting difference by another number (referred to as scale). Starting from an original variable (DNAmAge, PlasmaBlast, CD8pCD28nCD45RAn, CD8.naive), one arrives at a standardized version using the following formula Standardized.Variable=(Variable-Center)/Scale where Center and Scale are numeric variables which take the following values in case the input variables were estimated.

| | Center | Scale |
|---|---|---|
| DNAmAge | 10.398623 | 0.859736140 |
| PlasmaBlast | 1.515325 | 0.003915716 |
| CD8pCD28nCD45RAn | 1.540398 | 0.136369017 |
| CD8.naive | 269.536647 | -1.137203262 |

The following shows how to transform the variables.
Standardized.DNAmAge=(DNAmAge-10.398623)/0.859736140
Standardized.PlasmaBlast=(PlasmaBlast-1.515325)/0.003915716
Standardized. CD8pCD28nCD45RAn=(CD 8pCD28nCD45RAn-1.540398)/0.136369017
Standarized.CD8.naive=(CD8.naive-269.536647)/(-1.137203262)

### Step 6: Combine the standardized input variables into a single estimate of biological age.

The 4 standardized input variables from the previous step are combined into a single estimate of biological age (denoted by BioAge4 where 4 reflects the four input variables) by forming a weighted average as follows.

BioAge4=weight.1*Standardized. DNAmAge+weight. 2 * Standardized. Plasma Blast+weight.3*Standardized.CD8pCD28nCD45RAn+weight.4*Standarized.CD8.nai ve.

In case the input variables were estimated using the coefficient values such as the following weights.
weight. 1=0.931238800, weight.2=0.009650902, weight.3=0.041428925, weight.4=0.017681373.

### Step 4: Calculate mortality estimators

The BioAge4 measure from step 3 predicts life expectancy due to all cause mortality (Chen et al 2016). The higher the value of BioAge4, the higher the risk of dying early.

It can be convenient to use BioAge4 as starting point of other derived mortality predictor. For example, BioAge4 could be an input of other composite biomarkers of aging, i.e. it could be used as a component of a weighted average with other mortality predictors.

BioAge4 lends itself for defining an age adjusted estimate of biological age referred to as measure of age acceleration by contrasting BioAge4 with the chronological age of the DNA donor (at the time of DNA collection). For example, age acceleration can be defined as a regression residual resulting from a linear regression model that regresses BioAge4 (considered as dependent variable) on chronological age and possibly other covariates. For example, the measure of extrinsic epigenetic age acceleration, which is independent of chronological age, is defined as residual from a regression model that regresses BioAge4 on chronological age and possibly other covariates such as sex.

Another measures of age acceleration results by forming the difference between BioAge4 and chronological age, i.e. Delta.Age=BioAge4-Age.

### Applications

The measures of BioAge4 or derived measures such as epigenetic age acceleration lend themselves for identifying individuals at higher or lower mortality risk. This information has many applications including the following. For example, it can inform pricing decisions for life insurance policies (medical underwriting). Or it can allow one to assess the prospects of medical interventions (e.g. it can be used in clinical trials).

The complete list of 143 CpG markers that are used in this invention. See Table 1.

The segregated subsets of these CpG markers that are used to examine plasma B cells, naive cytotoxic T cells and exhausted cytotoxic T cell counts

### CpGs and coefficient values for plasma blasts.

| Probe | CoefPlasmaBlast |
|---|---|
| cg24735235 | -1.22037 |
| cg01372366 | -0.54864 |
| cg25521400 | -0.0983 |
| cg01124420 | 0.016947 |
| cg13553498 | 0.027385 |
| cg26164712 | 0.124548 |
| cg20244489 | 0.331334 |
| cg08945443 | 0.503181 |
| cg13608166 | 0.860483 |
| cg06245711 | 1.004191 |

### CpGs and coefficient values for exhausted cytotoxic T cells.

Note that CD8pCD28nCD45RAn denotes exhausted cytotoxic T cells defined as CD8+, CD28-,CD45RA- T cells.

| Probe | CoefCD8pCD28nCD45RAn |
|---|---|
| cg07094298 | -32.49636246 |
| cg04683740 | -28.78664576 |
| cg26655856 | -25.17478151 |
| cg14518178 | -24.35041137 |
| cg01372366 | -16.41198622 |
| cg09197075 | -15.27271944 |
| cg03132824 | -8.804874405 |
| cg00147638 | -8.585092039 |
| cg25020550 | -6.902339858 |
| cg23731272 | -5.103571211 |
| cg22513455 | -4.871530954 |
| cg00495443 | -4.110671958 |
| cg08688907 | -4.064796281 |
| cg21912203 | -3.894792915 |
| cg10688297 | -3.798663762 |
| cg10909506 | -1.876296791 |
| cg26091609 | -1.134175388 |
| cg00073460 | -1.004094248 |
| cg08454507 | -0.939981734 |
| cg20723792 | -0.583390023 |
| cg14969094 | 0.465448502 |
| cg01124420 | 0.625886381 |
| cg06445016 | 3.952559343 |
| cg05217983 | 4.731796544 |
| cg03467087 | 4.880162127 |
| cg02988775 | 7.018824197 |
| cg01345395 | 7.432856251 |
| cg16549957 | 7.505678912 |
| cg21593149 | 7.508381938 |
| cg00871371 | 10.37906776 |
| cg11685391 | 11.84617862 |
| cg23001918 | 12.97374972 |
| cg26485825 | 14.45361589 |
| cg08482359 | 16.60883764 |
| cg13608166 | 51.38004579 |

### CpGs and coefficient values for naive CD8+ T cells.

| Probe | CoefCD8.naive |
|---|---|
| cg15867698 | -285.528 |
| cg17478979 | -218.636 |
| cg25289028 | -179.36 |
| cg10909506 | -129.815 |
| cg23001918 | -124.964 |
| cg17820878 | -112.25 |
| cg07107916 | -106.563 |
| cg09025210 | -90.2945 |
| cg07899551 | -83.9632 |
| cg03370106 | -79.9871 |
| cg26485825 | -71.6959 |
| cg21097090 | -59.4431 |
| cg25130381 | -54.5947 |
| cg16662477 | -47.3162 |
| cg06952412 | -44.2347 |
| cg05392293 | -31.2567 |
| cg26720010 | -30.7259 |
| cg04683740 | -23.5495 |
| cg08945443 | -18.568 |
| cg20386303 | -18.4978 |
| cg15535471 | -12.5606 |
| cg25020550 | -0.13886 |
| cg24376214 | 3.230951 |
| cg21593149 | 12.30577 |
| cg25639084 | 21.31766 |
| cg15989436 | 25.45516 |
| cg02033323 | 35.3506 |
| cg18346531 | 44.4135 |
| cg02989940 | 52.64757 |
| cg10274029 | 58.27143 |
| cg07955474 | 67.83256 |
| cg18442362 | 87.38492 |
| cg23876292 | 118.8169 |
| cg12966876 | 140.7811 |
| cg17850367 | 156.176 |
| cg01372366 | 180.0996 |
| cg05913271 | 188.7031 |
| cg07094298 | 321.4028 |
| cg10493055 | 410.2807 |

Remember that the method optionally makes use of the DNAm age estimator by Hannum et al 2013 (Molecular Cell).

### CpGs and coefficient values for the Hannum estimate of DNAmAge.

| | |
|---|---|
| Probe | CoefDNAmAgeHA |
| cg05442902 | -22.7 |
| cg22285878 | -20.7 |
| ch.13.39564907R | -20.6 |
| cg09651136 | -15.8 |
| cg20822990 | -15.7 |
| cg06685111 | -13.1 |
| cg20052760 | -12.6 |
| cg02867102 | -12.5 |
| cg16054275 | -11.1 |
| cg00486113 | -10.7 |
| cg22796704 | -10.6 |
| cg02046143 | -10.2 |
| cg04474832 | -7.1 |
| cg08415592 | -6.92 |
| cg10501210 | -6.46 |
| cg13001142 | -5.8 |
| cg19722847 | -5.66 |
| cg04875128 | -4.37 |
| cg06874016 | -4.37 |
| cg19283806 | -4.29 |
| cg14556683 | -4.04 |
| cg03473532 | -3.31 |
| cg08234504 | -3.16 |
| cg01528542 | -2.98 |
| cg00481951 | -2.72 |
| cg22158769 | -2.06 |
| cg25428494 | -1.81 |
| cg16419235 | -1.6 |
| cg07927379 | -1.42 |
| cg09809672 | -0.74 |
| cg23091758 | -0.392 |
| cg23744638 | 0.0859 |
| cg02085953 | 1.02 |
| cg22512670 | 1.05 |
| cg22016779 | 1.79 |
| cg24079702 | 2.48 |
| cg07082267 | 2.87 |
| cg07583137 | 3.03 |
| cg07547549 | 3.11 |
| cg07553761 | 3.72 |
| cg25410668 | 3.87 |
| cg25478614 | 4.01 |
| cg22736354 | 4.42 |
| cg22454769 | 4.85 |
| cg23500537 | 5.67 |
| ch.2.30415474F | 5.79 |
| cg00748589 | 8.21 |
| cg23606718 | 8.35 |
| cg21296230 | 8.39 |
| cg03032497 | 8.4 |
| cg18473521 | 8.85 |
| cg06639320 | 8.95 |
| cg08540945 | 9.41 |
| cg06493994 | 9.42 |
| cg04400972 | 9.62 |
| cg02650266 | 10.2 |
| cg03607117 | 10.7 |
| cg14361627 | 10.7 |
| cg16867657 | 10.8 |
| cg04940570 | 11.6 |
| cg04416734 | 11.9 |
| cg06419846 | 13.4 |
| cg19935065 | 13.4 |
| cg07955995 | 13.7 |
| cg11067179 | 14.7 |
| cg21139312 | 17.1 |
| cg14692377 | 19.1 |
| cg20426994 | 19.1 |
| cg22213242 | 23.7 |
| cg08097417 | 27.3 |
| cg03399905 | 28 |

### ILLUSTRATIVE ALGORITHM ASSOCIATED WITH THESE MEASUREMENTS

The typical algorithm can proceed along 4 steps.

### Step 1: Estimate 4 input variables

i) DNAmAge is an age estimate based on the method by Hannum 2013 or by an alternative method. The Hannum estimator is based on forming a weighted average of the DNA methylation levels of 71 CpGs.
ii) PlasmaBlast is an estimate of the abundance of plasma blasts.
iii) CD8pCD28nCD45RAn is an estimate of the abundance of exhausted cytotoxic T cells defined by cluster of differentiation markers (CD8+, CD28-,CD45RA- T cells).
iv) CD8.naive is an estimate of the abundance of the naive cytotoxic T cells.

The three blood cell count estimates can be estimated using flow cytometric methods or DNA methylation data. To estimate the blood cell counts based on DNAm data, one measures the methylation levels of the respective sets of CpGs. Next one forms a weighted average of the methylation levels using the reported coefficient values and adds an intercept term.

### Step 2: Standardization of the 4 input variables.

Each input variable can be standardized by subtracting a value (referred to as center) and dividing the resulting difference by another number (referred to as scale). Starting from an original variable (DNAmAge, PlasmaBlast, CD8pCD28nCD45RAn, CD8.naive), one arrives at a standardized version using the following formula Standardized.Variable=(Variable-Center)/Scale where Center and Scale are numeric variables which take the following values in case the input variables were estimated using the coefficient values.

| | Center | Scale |
|---|---|---|
| DNAmAge | 10.398623 | 0.859736140 |
| PlasmaBlast | 1.515325 | 0.003915716 |
| CD8pCD28nCD45RAn | 1.540398 | 0.136369017 |
| CD8.naive | 269.536647 | -1.137203262 |

The following R code shows how to transform the variables.
Standardized.DNAmAge=(DNAmAge-10.398623)/0.859736140
Standardized.PlasmaBlast=(PlasmaBlast-1.515325)/0.003915716
Standardized. CD8pCD28nCD45RAn=(CD 8pCD28nCD45RAn-1.540398)/0.136369017
Standarized.CD8.naive=(CD8.naive-269.536647)/(-1.137203262)

### Step 3: Combining the standardized input variables into a single estimate of biological age

The 4 standardized input variables from step 3 are combined into a single estimate of biological age by forming a weighed average as follows.

BioAge4=weight. 1*Standardized. DNAmAge+weight. 2 * Standardized. Plasma Blast+weight.3*Standardized.CD8pCD28nCD45RAn+weight.4*Standarized.CD8.nai ve.

One can use the following weights:

| | | |
|---|---|---|
| weight. 1=0.931238800 | weight.2=0.009650902, | weight.3=0.041428925, |
| weight.4=0.017681373. | | |

### Step 4: Calculate mortality estimators

The BioAge4 measure from step 3 can be correlated with the chronological age of the DNA donor (at the time of DNA collection) and it predicts life expectancy due to all cause mortality (Chen et al 2016). The higher the value of BioAge4, the higher the risk of dying early.

It can be convenient to use BioAge4 as starting point of other derived mortality predictor. For example, BioAge4 could be an input of other composite biomarkers of aging, i.e. it could be used as a component of a weighted average with other mortality predictors. Or BioAge4 can be used to define a measure of age acceleration, e.g. as a residual resulting from regressing BioAge4 (considered as dependent variable) on chronological age and possibly other covariates. For example, the measure of extrinsic epigenetic age acceleration, which is independent of chronological age, is defined as residual from a regression model that regresses BioAge4 on chronological age and possibly other covariates such as sex.

Another measure of age acceleration results by forming the difference between BioAge4 and chronological age, i.e. Delta.Age=BioAge4-Age.

### REFERENCES

Note: This application references a number of different publications as indicated throughout the specification by reference numbers enclosed in brackets, e.g., [Chen et al., 2016 Sep 28;8(9):1844-1865. doi: 10.18632/aging.101020]. A list of these different publications ordered according to these reference numbers can be found below.
[1] Bocklandt S, Lin W, Sehl ME, Sanchez FJ, Sinsheimer JS, Horvath S and Vilain E. Epigenetic predictor of age. PLoS ONE. 2011; 6(6):e14821.
[2] Hannum G, Guinney J, Zhao L, Zhang L, Hughes G, Sadda S, Klotzle B, Bibikova M, Fan J-B and Gao Y. Genome-wide methylation profiles reveal quantitative views of human aging rates. Molecular cell. 2013; 49(2):359-367.
[3] Horvath S. DNA methylation age of human tissues and cell types. Genome Biol. 2013; 14(R115).
[4] Weidner CI, Lin Q, Koch CM, Eisele L, Beier F, Ziegler P, Bauerschlag DO, Jockel KH, Erbel R, Muhleisen TW, Zenke M, Brummendorf TH and Wagner W. Aging of blood can be tracked by DNA methylation changes at just three CpG sites. Genome Biol. 2014; 15(2):R24.
[5] Marioni R, Shah S, McRae A, Chen B, Colicino E, Harris S, Gibson J, Henders A, Redmond P, Cox S, Pattie A, Corley J, Murphy L, et al. DNA methylation age of blood predicts all-cause mortality in later life. Genome Biol. 2015; 16(1):25.
[6] Christiansen L, Lenart A, Tan Q, Vaupel JW, Aviv A, McGue M and Christensen K. DNA methylation age is associated with mortality in a longitudinal Danish twin study. Aging Cell. 2015.
[7] Perna L, Zhang Y, Mons U, Holleczek B, Saum K-U and Brenner H. Epigenetic age acceleration predicts cancer, cardiovascular, and all-cause mortality in a German case cohort. Clinical Epigenetics. 2016; 8(1):1-7.
[8] Horvath S, Pirazzini C, Bacalini MG, Gentilini D, Di Blasio AM, Delledonne M, Mari D, Arosio B, Monti D, Passarino G, De Rango F, D'Aquila P, Giuliani C, et al. Decreased epigenetic age of PBMCs from Italian semi-supercentenarians and their offspring. Aging (Albany NY). 2015.
[9] Fagnoni FF, Vescovini R, Passeri G, Bologna G, Pedrazzoni M, Lavagetto G, Casti A, Franceschi C, Passeri M and Sansoni P. Shortage of circulating naive CD8+ T cells provides new insights on immunodeficiency in aging. Blood. 2000; 95(9):2860-2868.
[10] Franceschi C. Inflammaging as a major characteristic of old people: can it be prevented or cured? Nutr Rev. 2007; 65(12 Pt 2):5173-176.
[11] Franceschi C, Bonafè M, Valensin S, Olivieri F, De Luca M, Ottaviani E and De Benedictis G. Inflamm-aging. An evolutionary perspective on immunosenescence. Ann N Y Acad Sci. 2000; 908:244-254.
[12] Miller RA. The Aging Immune System: Primer and Prospectus. Science. 1996; 273(5271):70-74.
[13] Marioni RE, Shah S, McRae AF, Chen BH, Colicino E and Harris SE. DNA methylation age of blood predicts all-cause mortality in later life. Genome Biol. 2015; 16.
[14] Horvath S, Pirazzini C, Bacalini MG, Gentilini D, Di Blasio AM, Delledonne M, Mari D, Arosio B, Monti D, Passarino G, De Rango F, D'Aquila P, Giuliani C, et al. Decreased epigenetic age of PBMCs from Italian semi-supercentenarians and their offspring. 2015.
[15] Baker G and Sprott R. Biomarkers of aging. Exp Gerontol. 1988; 23:223-239.
[16]. Peters MJ, Joehanes R, Pilling LC, Schurmann C, Conneely KN, Powell J, Reinmaa E, Sutphin GL, Zhernakova A, Schramm K, Wilson YA, Kobes S, Tukiainen T, et al. The transcriptional landscape of age in human peripheral blood. Nat Commun. 2015; 6.
[17] Sanders J, Boudreau R and Newman A. (2012). Understanding the Aging Process Using Epidemiologic Approaches. (Dordrecht Heidelberg New York: Springer).
[18] Belsky DW, Caspi A, Houts R, Cohen HJ, Corcoran DL, Danese A, Harrington H, Israel S, Levine ME, Schaefer JD, Sugden K, Williams B, Yashin AI, et al. Quantification of biological aging in young adults. Proceedings of the National Academy of Sciences. 2015; 112(30):E4104-E4110.
[19] Roberts-Thomson I, Youngchaiyud U, Whittingham S and Mackay I. AGEING, IMMUNE RESPONSE, AND MORTALITY. The Lancet. 1974; 304(7877):368-370.
[20] Levine ME, Hosgood HD, Chen B, Absher D, Assimes T and Horvath S. DNA methylation age of blood predicts future onset of lung cancer in the women's health initiative. Aging (Albany NY). 2015; 7(9):690-700.
[21] Marioni RE, Shah S, McRae AF, Ritchie SJ, Muniz-Terrera G, Harris SE, Gibson J, Redmond P, Cox SR and Pattie A. The epigenetic clock is correlated with physical and cognitive fitness in the Lothian Birth Cohort 1936. International journal of epidemiology. 2015:dyu277.
[22] Levine M, Lu A, Bennett D and Horvath S. Epigenetic age of the pre-frontal cortex is associated with neuritic plaques, amyloid load, and Alzheimer's disease related cognitive functioning. Aging (Albany NY). 2015; Dec.
[23] Horvath S, Garagnani P, Bacalini M, Pirazzini C, Salvioli S, Gentilini D, DiBlasio A, Giuliani C, Tung S, Vinters H and Franceschi C. Accelerated Epigenetic Aging in Down Syndrome. Aging Cell. 2015; 14(1).
[24] Horvath S and Ritz BR. Increased epigenetic age and granulocyte counts in the blood of Parkinson's disease patients. Aging (Albany NY). 2015.
[25] Horvath S, Erhart W, Brosch M, Ammerpohl O, von Schönfels W, Ahrens M, Heits N, Bell JT, Tsai P-C, Spector TD, Deloukas P, Siebert R, Sipos B, et al. Obesity accelerates epigenetic aging of human liver. Proc Natl Acad Sci U S A 2014; 111(43):15538-15543.
[26] Horvath S and Levine AJ. HIV-1 infection accelerates age according to the epigenetic clock. J Infect Dis. 2015.
[27] Horvath S, Mah V, Lu AT, Woo JS, Choi OW, Jasinska AJ, Riancho JA, Tung S, Coles NS, Braun J, Vinters HV and Coles LS. The cerebellum ages slowly according to the epigenetic clock. Aging (Albany NY). 2015; 7(5):294-306.
[28] Spiers H, Hannon E, Schalkwyk LC, Smith R, Wong CC, O'Donovan MC, Bray NJ and Mill J. Methylomic trajectories across human fetal brain development. Genome research. 2015; 25(3):338-352.
[29] Walker RF, Liu JS, Peters BA, Ritz BR, Wu T, Ophoff RA and Horvath S. Epigenetic age analysis of children who seem to evade aging. Aging (Albany NY). 2015; 7(5):334-339.
[30]Klemera P and Doubal S. A new approach to the concept and computation of biological age. Mech Ageing Dev. 2006; 127(3):240-248.
[31]Houseman E, Accomando W, Koestler D, Christensen B, Marsit C, Nelson H, Wiencke J and Kelsey K. DNA methylation arrays as surrogate measures of cell mixture distribution. BMC Bioinformatics. 2012; 13(1):86.
[32]Viechtbauer W. Conducting Meta-Analyses in R with the metafor Package. J Statistical Software. 2010; 36(3):1-48.
[33]Curb JD, McTiernan A, Heckbert SR, Kooperberg C, Stanford J, Nevitt M, Johnson KC, Proulx-Burns L, Pastore L, Criqui M and Daugherty S. Outcomes ascertainment and adjudication methods in the Women's Health Initiative. Ann Epidemiol. 2003; 13(9 Suppl):S122-128.
[34]Deary IJ, Gow AJ, Pattie A and Starr JM. Cohort profile: the Lothian Birth Cohorts of 1921 and 1936. Int J Epidemiol. 2012; 41(6): 1576-1584.
[35]Ferrucci L, Bandinelli S, Benvenuti E, Di Iorio A, Macchi C, Harris TB and Guralnik JM. Subsystems contributing to the decline in ability to walk: bridging the gap between epidemiology and geriatric practice in the InCHIANTI study. Journal of the American Geriatrics Society. 2000; 48(12):1618-1625.
[36]Lin Q, Weidner CI, Costa IG, Marioni RE, Ferreira MRP and Deary IJ. DNA methylation levels at individual age-associated CpG sites can be indicative for life expectancy. Aging. 2016; 8.

**TABLE 1: Listing of 143 CpGs Set**

| This Table provides sequence and methylation residue information (in brackets) for the 143 CpGs useful in embodiments of the present invention. Further explanations of these sequences can be found, for example, on the Illumina^{™} website, under Technical Note: Epigenetics - CpG Loci Identification (Search: "res.illumina.com/documents/products/technotes/technote_cpg_loci_identification.pdf "). Briefly, these 143 CpGs correspond to Illumina probes specified by so called Cluster CG numbers (see Table 1 in the Illumina^{™} Technical Notes). | | | | | | |
|---|---|---|---|---|---|---|
| **S E Q I D N O** | **Probe** | **Forward sequence with the CpG site marked with []** | **C h r o n o s o m e** | **Position (Human genome built 36)** | **S t r a n d** | **G e n e** |
| 1 | cg00073460 | AGAAGTTCTCCTGCCTCCAGCTGAGAAGATGATCAG ATTCTAGCTGCTCCTGGGGAAAGT[CG]GTACTCACA GCTGGACACAAACATAGCTTGCAGGAGGAAGAGTGT CAGAGCAAGAGACAG | 6 | 149848195 | F | Z C 3 H 1 2 D |
| 2 | cg00147638 | AGGGGAAGCTGGGCTGGGGGGACTGGCTTGGATGT GTTCTCAAACCATCTCTGCCAGCAG[CG]TGCTGGGTC CTGCCCCATCTGTACAATGAGGGGAACCCCGCTCGA GGGTGGTGGGGGTGG | 1 | 25100626 | R | R U N X 3 |
| 3 | cg00495443 | TATAGTGAGGGGAGGGAAGGCCACAGAGGTGGCCA GAGGACCTGGATTTGCAGGCAGCAT[CG]CACTTACT GTCTCTGCAGTCGGGCTTTGAGGCTGCCTGGGCCTG GGTTCCCTCATTTCCA | 22 | 15948163 | R | I L 1 7 R A |
| 4 | cg00871371 | TGCTCTCAGTTCCTAACAGAAAGTGTCGGGCCCCCAG TGGGATTTCTGGGGAAGAACTCT[CG]TGTCTCAACG GGAGCCCTGTGGCGGGAGGGGAGGCCAGGGTTTGG GGTTGTGTTCGTTGT | 1 7 | 72883071 | R | 9 S e P |
| 5 | cg01124420 | GGTCTATGAAGAAAGTCAGGTACCATCCAGTTCTGG GTGACAGGCTTCACAGGTGGCCAC[CG]TGGCAGCCC TCTCAGAAGCAGGTACTCTATGTCCTTAGGGGCCAGC AGGGCTGCCTGGGA | 2 | 108971950 | R | E D A R |
| 6 | cg01345395 | CCATTGAAGTGGGTCAAGTGTGTTGTCGAACGTAGT GCACACGTACAACGGCAGGTCTGT[CG]CACACGGAC GACGGCAGGTCTGTCGCACACGTACGACGGCAGGTC TGTCGGCACCTGAGG | 6 | 170367439 | R | |
| 7 | cg01372366 | AAACTTCAGGAGGTTTTAGCTAGTGGCGATCCAAAG ACATTAAATATTGCTTTGTGGGTA[CG]TCTCCACCTT GCGTCTTACTTGGTGACTGTTCAGTGGTAGGCAAATG TCAGAACCCAGTA | 11 | 47998359 | F | P T P R J |
| 8 | cg02033323 | AGAGCCAGAACCAGGGTCCCACCTAGAGTCCTGAGA TCTAGGCTTGGATTTCAACTCTGC[CG]CTGCATTTCG GTGAGGCCCTGAGATCTCTGGTCTTCAATTTGCCCTT CTACACTGAGCAC | X | 49005257 | R | F O X P 3 |
| 9 | cg02988775 | GAGTAGAGAGCCCTGGGTGGGGGTACTCACAGAGT AGCCGTGTGTGGCGGTGCAGGGCCC[CG]CAGTCTAG GCATGGTTGGCAGTAGAAGGGTGAACTGCTGACACA TTGGCTGACCTGGCAC | 1 | 6447229 | R | T N F R S F 2 5 |
| 1 0 | cg02989940 | CTATCTTAGGCTGACACTTGACTCCTTGCCTACATCTA TAGCTTGGCACAGAGAGATTCA[CG]CACCCTCAAGA GTGTGGGTGAGACATATACAGCCTGTTAGACCTGAA GGTGAGCCCAACC | 1 6 | 31446735 | F | A H S P |
| 11 | cg03132824 | AAGGTGAAGCTGCGTGAGGCTCGGGGAGCAGCTCT GGTCTTCTTTGGGGCGTGCCTGCCG[CG]GGCCGCCT CTGGCTGTCTTGGGGCAGGGGGAATGTGTTCCTGTA ACCTCTGTTTCATATC | 4 | 3342804 | R | R G S 1 2 |
| 1 2 | cg03370106 | AAAGGGTTCTTGGTACTAACCTCACTTTAATCATTGC CAAAGAGCATGAAGACAGCCCAG[CG]TCCTCCCACA AGAAAGCCTTGGGCCTCACAAACTTGGCAAGCAGTC TCAGGCACAAAACT | 3 | 11598800 | R | V G L L 4 |
| 1 3 | cg03467087 | CAGCCCCACCCCATGGCATAGCAGCAGGCCCCTGGG AGCCCTGAGCGCTCATTAAAGGAA[CG]CACTTTAAT GACAGCCACACGCCACCCTGCCACAAGCAGCGCTCTT CTCCCTATGAAGTG | 6 | 158368122 | R | 5 Y N J 2 |
| 1 4 | cg04683740 | TGCTCACATCTCTGCTGAGCCTCTCTTGATGGAGACC TCCTGAGCCTGGCTTCACTCTGC[CG]GTTCCACCTGG GTGGAATGCGTGTGTGGAAACAGGTGACGGGTTAA GCCCTTAGGAAGGT | 2 | 30315630 | R | L B H |
| 1 5 | cg05217983 | AAAGTTTTGCCTCTACCCAAGCAGCATACCTTCCTAG CAGTGGGAGCATATGGTGGGTCC[CG]TCCCTTTTGCT GCTGTCTCTTTTCACAAAGGCTGACTGCTTGCCTGAA CTAGGGAGGTTT | 6 | 45514845 | R | R U N X 2 |
| 1 6 | cg05392293 | GACCCAGGCAGGTAAGAGGCTGGTACAACAGGAAG CTCTCCTGAGGGCGTTATGGCATCT[CG]CTGAAGAG GAACTCCTACCCACAGCTCAGTTGTGTGATGTGGGG AGGTGGGTGCACTTTC | 1 0 | 13951337 | R | F R M D 4 A |
| 1 7 | cg05913271 | CTGGCCTCCTCCCTGCGCCTCCTGCGGGCCGACTGCC TCAGCACGGGCATGGAGCTGCTC[CG]GCGGATCCAG GAGAGGCTGCTTGCCATCCTGCAGCATTCTGCCCAG GTACTGCCCGGCCC | 6 | 43299066 | F | C U L 9 |
| 1 8 | cg06245711 | TTCTGTTAATTGATCTGGGTGTTCACTTGGTGAAAAG TCTGGAAGCTGTACCTGCGACAT[CG]CACTTTCCTAA TGTACGTTATATTTCAATAAAAGATTTTTTTAAATTGG ACTGCTTGAAA | 1 7 | 71772371 | R | F A M 1 0 0 B |
| 1 9 | cg06445016 | TGCACGTCGCCCTGGGAGTCGTTCCCGGGCCCCTCTG CAGACACAGCCTTCAGGTGTGAG[CG]GTGTGTGCGC CGGCCGGGGGCGTCTCTTACAGGAAGTTTATATTTGC AAACTGGGCGGTG | 8 | 61998402 | R | |
| 2 0 | cg06952412 | TTTGGAGGAGGCGCCTGGCCCAGCATGAGAAGAGG ATTAGGGAGTTCTTCCTGTAGGAGA[CG]ACATCTGA ACTGAGAGCTGTGGGATGAATAGAAATGAGCCAGG CCCGATGAGTGAGGAGG | 1 9 | 45817690 | F | L T B P 4 |
| 2 1 | cg07094298 | TAATTTTTGAGAATTCCCCTCAAATGACAAAGGAAGT GCACGTCCTAACACAGCCTACAC[CG]GGAAGCCCCG GGTGACTCAGCTGAGTTCAGGGCTGAGAAGTGACAC TTATTTAGAGACTG | 4 | 2717824 | F | T N I P 2 |
| 22 | cg07107916 | TGTCAGAAACGCTGCTGAGGTTTGAACCTCCTTGACT GGGAGGATTATGTACAATTTCAC[CG]CCCAGCAAAT CTGAAGGAGGAAATGTTTTGAGGATAGTATGTTTGG TTCTAGGTCTAGAA | 1 | 210524047 | F | P P P 2 R 5 A |
| 2 3 | cg07899551 | CCAAAAGTGTTACATGGATACACACAAGGCCCAAGC AGCACCACAGTTAGAATGCCGGGT[CG]TGTGGCTAT GAGACACAGGCCTGCCACAGAAGCCATGCCTGGCTT CTTGAACATTCTAGG | 1 | 206101847 | R | |
| 2 4 | cg07955474 | AAAGAGGTCACATTTTAATCAGAAGAACAAAAATGC TATTTTTTTTTTTTTTTGGGTGGG[CG]TCTATCCAGCT GTCTTGGGGATTTCCAGCACTGTTCTTGGAAATGTTG GCTCTAAGTCGA | 1 6 | 84493057 | F | I R F 8 |
| 2 5 | cg08454507 | ATATTTTTAAGAGTACATTTTGCCACGCTGTCTCGCGT GGGGCCTCTGCAGCAGCTGAGA[CG]TTTGGCAGTTT CTTCAAGACTTGGAGTAAAACCTCGCCTGAGATAGA ATCTAAATACACT | 1 7 | 76370001 | F | R P T O R |
| 2 6 | cg08482359 | GCCGACAACGTCGTGTGTAGACTCAACGGCCCTGAA ATGCGCTTCCGGGAAAGGTCAAAA[CG]GTCGGCGTG CTGTGACGTGCATTTTACCACAATGGAAAGACCCGCA CGCTCCTTTCTCGA | 7 | 589360 | R | P R K A R 1 B |
| 2 7 | cg08688907 | CTGCATATTGATAGTGGCAGCGTAATGTGATCCTTTT CCCTTGCGCAATGCTTTAGATAG[CG]CTCTGGATGTC CAGCTTGGGAAAAGGCCACATGAGACACACAACAAA GATATTTAGTAGG | 5 | 159823503 | F | |
| 2 8 | cg08945443 | ACACTGCCTGGGGTGATAAATACTTTACTCAATTATA AACTAAGCAAGCAGACAGGTTGA[CG]GAAAAAAA AACTTACCCTGCTAAGTCCTCAGCTTCCTTCCTTCTCTCT TTCCTCCAAGTG | 1 0 | 74863260 | R | Z M Y N D 1 7 |
| 2 9 | cg09025210 | GAAGACCCAGCCGGCCGAGAGCCTCAGCCACCTTCC TGCAGGAGGTCCTCACACCCCAGA[CG]GTCAGAATG CTCCCCAGACTGAGGAATCAGCTGCACATCCCCCTGA TGTCTCTAAAGCTG | 3 | 142614836 | F | Z B T B 3 8 |
| 3 0 | cg09197075 | TCACCTGCCTGGTACAGCAGCACTCGGGGTTTCAAA GGTCATGAACAGGAGCTGGGTGAG[CG]TGGGTGAG CAGACGCCTTCGAACGCCGGCCAGCATGCTTTTTGTC AGGGTGAGGGTGGGA | 1 6 | 11677192 | R | S N N |
| 3 1 | cg10274029 | CGGGTGGCTCAGGAAACTGCGACCCTTTCTGGGACG GTGCTGTCCCCCGAGTGAGACCAT[CG]AGAGGGCAC TCCGCGTGTAGAGCATTCCCGTCTCGCGCTGAGGCG CGGCACCTGTGGCCT | 1 6 | 84538837 | R | |
| 3 2 | cg10493055 | TGAAAATTAGGAAACCATGCTGTTGGCAATCTCTTCT TCATAATTGTATGGGAGTAGCAC[CG]AGTCTGCTGTT TTTGGCAATCACAGACAGGTTGGTGACTCTGTAGAG AGCAGAGGTGTGT | 4 | 99795831 | F | T S P A N 5 |
| 33 | cg10688297 | GTCTGCAGTCACCTTTGACTCTGATGTGGAGACGGTG AGTGCCGCTGGGTGTCAGCCGTG[CG]ACCTCGGGCT GGTTGCGCTGCCCTTGCAGACTGCACTCGGAAGCGT AAAATAGGCCGTTC | 7 | 2573350 | R | I Q C ε I Q C ε |
| 3 4 | cg10909506 | GTTTCCTCTTCTCAGACAGCTCCAAGCTTAGAGACCT CAGAGAAAATGTGACCCACAACA[CG]CCATCCCAAC AAGCAAACACCCGTGCGCCAGCCTGGGGCGCATTCT GATTGAAGAGATGC | 1 7 | 35335521 | F | O R M D L 3 |
| 3 5 | cg11685391 | CCCCATCACCCCGCCTCTGCCCCACAAGCCTCCCAACC ACAGCCGCCGCCTCTTCCTCTT[CG]CAGGGCCACCTC AGCCTTTGGACTGGCACCTCCTTGGGAACTTTTGGCC AAGATATGGGC | 1 0 | 103584324 | F | K C N I P 2 |
| 3 6 | cg12966876 | TTAATTACAAACTTTGAAATCTTGGTCAGTGAAAGTT CATTTTAGTGCATCTTGCAAATA[CG]ATCAAAGGTCT TTTCAGTACTGATTTGATGATGCTTAACCACAGTGTG TGAAGAATGAAG | 2 | 111590321 | F | A C O X L |
| 3 7 | cg13553498 | TGAGAGCCAACTCCTTCCTTTCAATAAATTCTTATTTT CTATCATAATATTGCTTTGTAC[CG]CACAAAATACAA TTATAATTTTTAATAGTCAAAGAAATTTTAAGTTATCA AACATTAGTT | 1 2 | 9713253 | R | C L E C 2 D |
| 3 8 | cg13608166 | ATGAGGGCTGCAGAACTGGGCCAGCCGGAAAGTGT GATCTAGTCCTTGATACACACAGGC[CG]GCAGCATG GGGACACGCGTGGTTTCCACACCAGCCATCGCTGAG ACCACCGGGAAGAACT | 1 8 | 75258974 | R | N F A T C 1 |
| 3 9 | cg14518178 | AAGAAAAAAAAAAGAAAAGGTCAGCTGAGCAAAGT GTGTAAGGGGCAAAGTGATAAGAGT[CG]AAGTTGG AGAAGTAGGCAGGCGCCAGATTATGCGACACTTTGT GGACCACAGTAAGGAGG | 1 7 | 27792155 | F | |
| 4 0 | cg14969094 | TTGCCTTTTGTATGCCTTTGATGTGATTGCTCAGCTTC CAAGGGAGGGGAACGAGTGTCT[CG]TCTCTCCCACC TAGAGGCTCGTGCTGCCTGTGGTCAGGAAGAGAGCG AACAAAGGCGTGC | 3 | 158330697 | F | |
| 4 1 | cg15535471 | GTGTCATTGTTCACACTCATGAGCTGGAAGAAGCTGT GGTTTCATTATCTTCTTTCCTAC[CG]CCAAGCACTGTG CTAAGAACAGTAAATACCCAATACACAGATGTTTGCT GAACTGAGTAG | 7 | 138982627 | R | H I P K 2 |
| 4 2 | cg15867698 | ATGGCTCCCTTTAAGAGTCAAGGGTGCCAGCACCTTC ATTTCCAGGCCTCTCTCGCTGTG[CG]GCTGTGAACAT GTTGTTCTCTATCTCTAAACTCAGGATCTTCATCTATG AAGTGTCATCA | 1 4 | 68508020 | R | A C T N 1 |
| 4 3 | cg15989436 | TTAAACTAAAATTAAATAGAATTTTAGTCCAGATTTTC TAAGTGATCAAAGAGGAAGCTG[CG]TCAGCTGTGTC TGCCTGCCCTTGGCCCACTCCCTGTGCCCTGGGCTCT CTGTTACAGGTT | 5 | 150446068 | F | |
| 44 | cg16549957 | GGCCCATTCCAGGGGAGCGGGGCAGCAAGGTGTGA GACAGGCACCGGGCAGGCGCAGCAC[CG]AGTAGCA GCAGCAGCAGGTAGAAACAATAAGCGGATCTGAACT CATCAATATGGAAAGTT | 1 | 12141520 | F | |
| 4 5 | cg16662477 | CTAATTGCTGTGGTTTTTGGATGTTTCTGATAGCAGG CACTGCTTGCTGAGCTAAAACTG[CG]GTGAGAGTTC ATTCATTCTGGACTGGTTACTTCTGCAGCCGACATATT TGTAAAGACTTA | 1 7 | 30425543 | F | R F F L |
| 4 6 | cg17478979 | GCCCACGGGTCGTCGGGTGGCCTGCGCGGGGAAAG CAAGTCGCCGTGCAGGTCCCTAGGG[CG]GTGTTCGC CCCGCGGCTGGAGCTGCAGGCCGGGCGGAGGCGGG AGGTCGCCCGGGGAGAA | 6 | 149813843 | R | Z C 3 H 1 2 D |
| 4 7 | cg17820878 | GAACCGCCACGGGGTCCACGGCCGAGATGATGCTGC CGAAGAGCAGGTTGTCCAGGAGGC[CG]ATGTTGTTG ATCTGCTCACCGCCCACCAGGCACACGGCGTACATGA GGCCGCCCAGGAAG | 1 | 27313050 | R | S L C 9 A 1 |
| 4 8 | cg17850367 | CCCATCAATTCATTTAAAATTTAGCAAACATTTCTTGA TTGCCAGCTGTTTACCAAGCAG[CG]TACTAGACACTG GGGGTACAAGTTGAGTACGAATACTCCGTACCCTTAA AGACTCAATTA | 1 0 | 27190504 | F | A B I 1 |
| 4 9 | cg18346531 | TGCCAACAGAAGTTCAAAGATGCTCCGATAATGTGAT TTGTAAACACGAAGTCCCAGCAG[CG]GCGGCTGCTG CAGCTTCTTGGCTCTCTCACTCAATCCCCTACACCTTC TGGGGCCTCCCA | 1 7 | 9908156 | R | G A S 7 |
| 5 0 | cg18442362 | AGAATTAACTGTGTGTAACTGTATATTTGAGGCAAGG CAAGGGGACAGATATTTTCCTTA[CG]TTATTAGTTGT GCAACAGAAGCCAATTAAGAGATTGGAGAGATGAAT AACACTAGTGATG | 7 | 44644297 | F | O G D H |
| 5 1 | cg20244489 | TGAGCCTGTAATTTTTGTGCATCTTGAGGTAAACCCT GCTGAACTCTTTACCTAACACAA[CG]ATGCTTGATCC ACACCACATTCAGAATGCACAGGGGATAGGCTGGTC CCTTAACATTGTG | 1 | 8132374 | F | |
| 5 2 | cg20386303 | CCCACCCACAGGGCTGTTGATGCTAGCTGGGGGAGG GCACTGGCCCATCGTGGCAGAGGA[CG]TGGTCATCT GGGACCACTGCTTGTATAGCTCACTTATGTCCTCTGG TCCTGTTCAGGCTC | 2 | 238015280 | F | |
| 5 3 | cg20723792 | ATGGTACATAAATGGGCTCCTGTCAGCAGGAGAAAT TCTCTACTGATGCAGTGCCTGGGG[CG]ACAGAGGCT CATGCTGCCAAAGGCGAAGTGGGCTAAAGTAAAGGC TTCACACTGATGGAC | 1 0 | 126350659 | F | F A M 5 3 B |
| 5 4 | cg21097090 | ACCACTCAGATGTCGATGAAGCGAGCTATGTTGTGT AAATAACTCAGTTATCTTCTCTAT[CG]TTACTAGTCCT TTTCCTCTTATACGTAGCTTTTTTTTTTTTTTTCATCCTG GTGAACTTA | 5 | 118721663 | R | T N F A I P 8 |
| 55 | cg21593149 | TCTCTGTGATGTTCCTGTTCAAACCTTTTCCCCATTAA AAAAATGGGATTGCTAACCGGG[CG]AGGTGGCAGG CACCTGTAGTCCTAGCTACTCAGGAGGCTGAGGCAG GACAATCACTTGAT | 4 | 1290163 | R | M A E A |
| 5 6 | cg21912203 | ATCTTTAATACCACAGCCATTTCCTGAAAATGTATAGC TTTGCAAAAGTCTTGTCACTTC[CG]AAGGCTTCAGAA ATGCTGATTTGGAGGAGTTCCTATCATTAGGAATGG GACCCAGGTAGT | 1 | 64775891 | F | C A C H D 1 |
| 5 7 | cg22513455 | GAAGTGTTGCGTGGCCTGCCTCTTCCTTCATGAAGCC ACAAAGTGCCACTGCGTGAGTTC[CG]TGGCACATTG GAACACAGTGTACCCGCCTCTGGGCAAGAGCCGTGG AAATCCACACATGA | 3 | 120438534 | R | B 4 G A L T 4 |
| 5 8 | cg23001918 | GGCGGCCGGGAGGGGCTGGGGAAACCCAGGCGCAA GCGCGCTGGGCTGGGAGGACGGGGC[CG]GCCACCT CACCCGGCTCTCCAGCACCCCATTTTAAGGATGGGAC CACCCTCTGCCAGCCT | 6 | 392226 | R | |
| 5 9 | cg23731272 | GGGTGGGAGATTCCTGCTGTTAAAAACAAACAAAGG GCTTAGACACCGGCTAGTCGGCTG[CG]GGAGGCAGC AAGAGAAAGGCCGAGGGGCCTGGAGTAGCTCCTGA AAACCGCCGAGGGCCC | 1 5 | 65143892 | R | S M A D 3 |
| 6 0 | cg23876292 | GGCTTTAATTTACTGGTACATTATCTTGACATTTCTGC AGCAGAATAGATTAGTTGGTTC[CG]GCTCTGTAAAG GAAGTCAAGGTAGCTGATTTGACTAGTTGATTAACAT TCCTAATTGGCT | 1 8 | 55175497 | F | L M A N 1 |
| 6 1 | cg24376214 | GGAAGCCCCTCCCCATCCCCTGCCTTTCTTAAGCAGG GTGTGACCAATATGACACGTTGT[CG]AGTTTCGTCCA AACTGCTGTCTGTGCGTTTAAGCACATCATATAAACA TGTGCTCCTAGA | 1 9 | 43609975 | R | R A S G R P 4 |
| 6 2 | cg24735235 | AAATCAAAATGGTTTTAAAAGGTGTCATTTTCATTGG AACGGGGTGGGGCTCCTAGTGCC[CG]CCACAGTCCT CCACTCAGGCCGGCGGGGCGGGCCCTGGCCTCGGCC TCGGTGCGTGGGTG | 1 | 16036066 | R | F L J 3 7 4 5 3 |
| 6 3 | cg25020550 | ACGTGTCAGAAATAAGAGACTTCTCACACACACTTGA AGTATCTCTCCATGAGTGTTTAC[CG]CTTCCTTTGTGC AGGGCTTTGAGTCCTGTGTGGCATCAGACAGGCCTG TCTTAAGTTCCT | 8 | 134098140 | R | T G |
| 6 4 | cg25130381 | GCAGGAAGGGGGGTGTCTCGCCTACACCCTTGATCA GGCCCCCCACCAGCAGCCCCACCA[CG]ATCAGCAGG CAGCTCTCCGGGACGATGCTTGAGATAGTGGGGATC ACATGGAAACCTGCG | 1 | 27313308 | F | S L C 9 A 1 |
| 6 5 | cg25289028 | TAAGGCTGCTGAAACCTCAAGGTGGGGACGGGCTG GGAGGAGCATTGTGGCTGCGGGTTA[CG]GTACCAG GTACCCTTTGACACTCTTGGGGTTTCTCCAGAAAAA AAGACATTCCCCCATGG | 1 9 | 41120359 | F | L R F N 3 |
| 66 | cg25521400 | CTTCTTACGCAATGTAACTTCCTGCTGAAGAAAGGAC ATTGGAATTGGGTTTTCTGTCTG[CG]ATGGGAATGTT TAGTCAGATATTTAGAGAGCTTTTTCCCACAGATATTT TTATGGGGGTC | 2 | 62298783 | F | B 3 G N T 2 |
| 6 7 | cg25639084 | AGCAGCAGAAACGCATTTCACGTATTAAATATCACAC TGAAGTAGCACTTAAGATACCAA[CG]AGAAGGTTAA ATAAATAAGAGAGAAAGAACACCCTTGGCAGAAAGC ACAGCACGGCTACC | 1 3 | 23031513 | F | |
| 6 8 | cg26091609 | CTGGAATAGACATGACAATGGCTGCTGCCAAACTGTT TTCTCTTCTGAGGACAGATGTCC[CG]TGCAAGTGGCT TGGTGGAAGGGACTAGTGTCTCTAATATAGGGTGAT TTATAAGCAGGAA | 2 | 204442426 | R | C T L A 4 |
| 6 9 | cg26164712 | GTCGCGGCCCTACTGCCTCAGGAGACGATGCGCAGC TCATTTGCTTAAATTTGCAGCTGA[CG]GCTGCCACCT CTCTAGAGGCACCTGGCGGGGAGCCTCTCAACATAA GACAGTGACCAGTC | 1 1 | 118259775 | F | C X C R 5 |
| 7 0 | cg26485825 | TGTTATGTGCGCTCTGGCACAGGCTGACTCATGTGTG AAGTTTAAAGGTGGGGCCCCTGC[CG]CAGACAGCCT TCCTCACCTGAGTCAGGCAGGCCCGGGCACTGAGCA GGAAGGGCAGGTAT | 1 8 | 19706893 | R | L A M A 3 |
| 7 1 | cg26655856 | GTGGGGTCACAGCAAACAAAAAGGCTTTCGTAAACT CAAAAGCCCTCTGTGGCCCTGCCA[CG]GGGACGTCA TCTCTGGCGATCTGAAGGACAGGGGCAGGGAGGGA ACGGGAACCTCAGGCG | 1 6 | 87505388 | R | C B F A 2 T 3 |
| 7 2 | cg26720010 | ATGGCTCTGTCCTTTCCTGGGCCAAGTCTCTCAACACT GAACCCTCAGGTTCAGTCTCTG[CG]CTTATGAATGAG AAGCAGAAACCACACCTTCAAGGAAGTGAGCTGCGA ACAGAGAAGCTG | 7 | 45113656 | F | T B R G 4 |
| 7 3 | cg05442902 | GCCAGGTCACCCTCTCACTCTGTGCCTCTTAGTTATCT TGCATGCTCTGGTCTTTGCATA[CG]CTGCTCCCTGCA CCAGGAACCTCCATCCCCATCTTTGTCTGCTTGTCGAA CTTCAGAAAT | 2 2 | 19699010 | R | M G C 1 6 7 0 3 |
| 7 4 | cg22285878 | CTCCGCGTTCTTTCTTCTGCCGGCGAGCCAGGTAATG GTAACAGAGCGAAACTCCCCAGT[CG]GAACTTCTGG GTTGCAGCAGCCTCGCCCCCCCTCCCCGCAGCCCGCC GCCCGCTGGTCCG | 7 | 130069713 | R | K L F 1 4 |
| 7 5 | ch.13.39564 907 R | GTGAATGGATTTGGGGAGGGAGGATCAGACTAGAG GCCAGAAGTCCGGTGAGCAATATTA[CA]GTAGTCCA GGTGAGAGGTAATAATGGCCTGCCTAACCCAGGGAA GCAGCCACGGGGATTA | 1 3 | 39564907 | F | |
| 7 6 | cg09651136 | CCTGAGCTGAGGACAAACTAAGAACAGTGGTGGACA ACACTGGGATGGCAAGGATCTGGC[CG]ATGCTGGAC TATATTGTATTTTAAAGTTCTAAATTGTCTGGGTGCA GTGGCTCACGCCTG | 1 5 | 70312066 | F | P K M 2 |
| 77 | cg20822990 | AATGCCTGCTTCACAGAGAACTGCTGCGAGGATCAC ACAAGAAAATGCTTGTCAACTGGG[CG]TGGTGGCGC ATGCCTGTAATCCCAGCTACTCGGAGACTAAGCCAG GAGAATCGCTTGAAC | 1 | 17211353 | R | A T P 1 3 A 2 |
| 7 8 | cg06685111 | TCACCACTTCTTTGCCAGTCTAGATCCGTCCTGGTGCC TTACTGTGCATACAGTTCTACT[CG]TCTCAGGTGAGG AGGCCACTTAATTTGTAAAAGACTGAGGAAGGGGTA GGATCACCACAA | 6 | 30403445 | R | H C G 1 8 |
| 7 9 | cg20052760 | CTTGCGCCTCGAATGCCACGTTGAATACTCCTCATGT CTTTGGAGACATGTCCTTCCCTT[CG]AGCTGCTCCCA GTCAGGTGAGGAATAAAATGCTATGATGGCGTGAAA ATTCTCCCTTGGT | 6 | 10618775 | F | |
| 8 0 | cg02867102 | CTCCTGGAGTGGGTGCTCCTGGGATGCTTCAGGTTTA GACACCGGGGTTACGGCAGCTGC[CG]AGGAAGGCT AAAGCCAGCGTCCTGGATTCAGACAGACCTTTTAGCC ATTAAATCCACTAA | 1 7 | 59752425 | F | |
| 8 1 | cg16054275 | TTCATGAGCCGGACAAAGCTGTATCCCTCCATTTCCA CCTGCCAACACCACGGAAGCAGT[CG]TCCGTTACCAC TGACCTGAGGCCTGCCTGGGTCCAAGCTCACACTTG GAGAACCTTCTGT | 1 | 167822646 | F | F 5 |
| 8 2 | cg00486113 | ATTCACGGGGAATCAGAGGGTGGAGAGGGCGTGGG TGCCTGGAGATGCCTGGGAACAGAA[CG]GCTGAGG GGACTCCATTATCTGTACTCTTCCCGGGGTGGGTCTA GGTCTGGCTCCTCCTG | 6 | 31213690 | F | P S O R S 1 C 1 |
| 8 3 | cg22796704 | TCCTAAGCCTCTCTGAGCTGGGCTTGGCCACCTTCCG GGGTGTGAGCGTCCACGGGAGAT[CG]ACCACACCA GGCACCCAGGAGCAAGTGCTTTGAAATGCGGCTTTC TCCGGACCTTGCAGG | 1 0 | 49343540 | F | A R H G A P 2 |
| | | | | | | 2 |
| 8 4 | cg02046143 | GAGTCAGTGCACAGAGTTAGAGGCAGGCAGAAAGA AAGGAGACCTCCCCAACCTCGGCCC[CG]CCCTTCCCT GGCCCAGGCAACAAAGCAGCGAGGAGCCACGTGTG GAAAAGCAGTGCAGAT | 11 | 133303121 | F | I G S F 9 B |
| 8 5 | cg04474832 | CCAGCCAAGTGGCCTTGATCGTTTTCCCAATGCCCCC GAGCCTGTTTCCTGCCAGTAGAG[CG]GGTCAGATGT TGCCAACCTCTGCAGAGTAGCAATAAGCAGTAAACG CCACGCTCTGCACA | 3 | 51983527 | F | A B H D 1 4 B |
| 8 6 | cg08415592 | AGTATGTCAGTGGCAGGTCTTTCTCCTTGAGACCACA GCAGACCCCCAGCCCTGAGGATG[CG]AGGCAGGTG GGTTGGATGAGAGGGATCTGGATGTCTGGTCTCAGG CTGCTCCTCTAAGGG | 22 | 34978919 | R | A P O L 1 |
| 8 7 | cg10501210 | ACGTGGGGGAAGAAGGGGGTTACGCCATCAAGTCCT GAAGCCCGTCGGACCACCCATCGC[CG]CCTGCGCAG ACCCAAATCTTGGTCCCGCCGTAAGGTGCCGCAGTCC CGAATGTTCCAGAA | 1 | 206063643 | F | |
| 88 | cg13001142 | TGTGTTACTAAGTGAAGTCTACTATACATAGTAAATA CTACGCAGTCATACCTTTATAAA[CG]ACAAAATCTGA CCAGGCTAGATCTGAATCTGTTAATTTCTGAGACGGT GTGTCTGAAGTG | 6 | 147570214 | F | S T X B P 5 |
| 8 9 | cg19722847 | TCTGCTTACAGCTGCTTCCAAATTAAGCATATCTGGA TGGTGTGACACTTTTTGTTAGTC[CG]AGAACTGTATG GGCATCGCAACTGGGCCTGTTCCAAGATAGACTTGTT GGGACCTTCAAA | 1 2 | 30740381 | F | I P O 8 |
| 9 0 | cg04875128 | CGGCGCGCGCCGGGCTGTAGCTCTGCGACGACAGCG AGCGGTTCTGCTGCGGGTACGTGG[CG]CACGGCCGC AGCGCCCCCACGGCCGGCGCGCACGCCTCGTCCCGC GCGCCCGACGCCTGC | 1 5 | 29563187 | R | O T U D 7 A |
| 9 1 | cg06874016 | CAGCCTCTCAGGAGCTGACAGGTCCTCTTTCGGGGCT CAGGAGGGTGGGCACACACCCAG[CG]GCCTGCAGA GTAAGCTTATTACCCACAACTGTGCCCGCTTTGTGCTT CTAAGGTGCACAC | 1 7 | 37430941 | F | N K I R A S 2 |
| 9 2 | cg19283806 | TCCGTAGTATTGTCTCTGGCTTTGAACGCTGTTGAGG GAGGGGAATGTTTGCACTCATCC[CG]CATCCTTTTTT GGCTGCTATCTTTGCGGGGATTGTTCAAGGAGAAAT CCATCCTGACTGG | 1 8 | 64540400 | F | C C D C 1 0 2 B |
| 9 3 | cg14556683 | ACCAGCGCCACCGAGAACACCAGGCTCCACATGAAG GCGCGCAGCAGCTTCAGCGACAGG[CG]CGACGGCG CCAGCAGCGCGGTCACCACCAGCTCCGGCATGTCGC CGCGCTCCGGGACCAC | 1 9 | 15203982 | R | E P H X 3 |
| 9 4 | cg03473532 | AATTAAAGACTAATTCAGAATTTTCAAGTGATAGTAA ACAACTGCTATCTCAAACACATA[CG]ATATAAAATGA AACCACTGGTGCCTAACTGCCAGTTCTTTCACTCAAA CCTCTGCTGTGA | 7 | 130659283 | R | M K L N 1 |
| 9 5 | cg08234504 | TTGTATTTCAGCCCAAAGCCTACTGGAAGTGTCAAGC TGCCAGCTCCCCTCTGCCCTCCC[CG]TTGCTATGGCA GCCATGTCTCTGTGTGTGAATAGGTGAACCAGGCTCC AGGTTAGGACCT | 5 | 138993501 | F | |
| 9 6 | cg01528542 | TGTTACAATTTAACTACTTTCTCTTTCTCTTTCTCTCTCT CTCTCTCTCTCTGGTAAAAA[CG]TTAACCTCTGCTAGT GATGACCAAACCTGGTAAAGATTGTAAAGTGGGAAA AATTGGATT | 1 2 | 79992363 | F | |
| 9 7 | cg00481951 | TCAGAGGTCTGCGTTTCAGCACCTGGGTCAGCGCTTC CCAGGGTCAGCACCAGGGATAGA[CG]CCATTGTCGC TTCGTGCCCAGACAACTACCCAGATTTTGTAATTTTCT TGAATGCCAAGT | 3 | 188870344 | R | S S T |
| 9 8 | cg22158769 | AGAGCGCTACGTCGCCGGCGGGCAGCAGCAGCGCCT ACAAACTGGAGGCGGCGGCGCAGG[CG]CACGGCAA GGCCAAGCCGCTGAGCCGCTCTCTCAAAGAGTTCCC GCGTGCGCCGCCAGCC | 2 | 39041043 | F | L O C 3 7 5 1 9 6 |
| 99 | cg25428494 | GGGAGGGTGGGAGAGGGATGAGGGATGAGAAATT ACCTATTAGATACAATGTACAACATT[CG]GGTGACAG TTACACTAAGAGCCCAGATGCCACCACAATGCAATAT ACCCATGGAACAGAA | 4 | 84474435 | F | H P S E |
| 1 00 | cg16419235 | CTGCGCCCTCTGCAAAGGGCTGATTTCTACAGTCGCT AGGACCTGCAGCGGCGCTGCTCC[CG]CGGGGCTCCG GCCGCGCTGCATGTCCCATTATAGTCGCTAGAGGGC AGCGCTCTCCTGCG | 8 | 57523167 | R | P E N K |
| 1 0 1 | cg07927379 | CGTGGCTGCGCCCACAAAGCCGCCGGGGGCTGCGG ACTACAGCGAAGCCGGCGCGGGGCT[CG]GCCCTCAC TACACGAGGCCTGGGCGCCTGCACGCCCCCGTGCTT CAGCCCGCGGCTCCCG | 7 | 156125869 | F | C 7 O r f 1 3 |
| 1 0 2 | cg09809672 | CCCCAGAGAGCTTTCATCTAGAAGGTTTGACTCTGGC CAGACAACCAGCGAGCATCTTCT[CG]CAATCTGTTGC TTCTTCCATGGCAAACTCCAGAGAATTAAGAAGCCAA ACTCAACATCGC | 1 | 234624305 | R | E D A R A D D |
| 1 0 3 | cg23091758 | CAAAGCCGGCGAGGAGGCGGCGGCGCTGGTGGGGA CTGACCCGGCAGTCCGAGAATCCAC[CG]CGGCCTTTT CACCCAACCGCCCCCTCCTGCGTGGGGGCCCCGCATC CCCTGGACTGGCGT | 11 | 8982343 | R | N R I P 3 |
| 1 0 4 | cg23744638 | TCGACTTAAACCCACGAAGCTTTGGGGAGCACTCTAG CCCCTGCTACTCACCCATGCAAG[CG]GGGTGCGCGC TCGCGCACACACTCACTCACTCCAAGATAGGGGCTTT CTAGGAAAATACT | 11 | 10280478 | R | |
| 1 0 5 | cg02085953 | AGTTTTGCCTCCAGGGAAACTGAGGCACAAGGCAGC AATGATTACTGAGGGTCCTGCCTC[CG]CTCCTCTAGG TGAGGAGCCTATTCCAGGGGCTCCAGTCTGAAAGCC TAGAGGCGAGGGGC | 2 | 96565987 | F | A R I D 5 A |
| 1 0 6 | cg22512670 | GGGTCCTGATGGTGGTAACAACTGCTGATGCCCATCT TGGCCTGGGGCACTGAGATCGCC[CG]GAGATCACAG TGTTAGCTTCAGGGCGGGGTAGAAATTAGAGGATAG GGGATCTCTAGGGC | 1 | 26728352 | R | R P S 6 K A 1 |
| 1 0 7 | cg22016779 | CTACAGTGCCCGCAGGCCCCTTAAAAGGGAGCCAGT GCAACAGATTGGTTAACCAGGCCA[CG]TGTTCCCAG TCTTCTAAAAATCCCCAAAGACTGGACAGCAAATGCC CCTTAGGTTGCATG | 2 | 230160555 | R | D N E R |
| 1 0 8 | cg24079702 | CTCCGGGTCTTGGGAGCACAGTAGTTATCGGGAGCG TCGCCTCCGGCGTGGGCTCTCGGG[CG]CGAGTTTCG GACGAGGCCTGGGCGCGGTGGCAGGGGTCTGCCCA CGCCGGGATCTCTGCC | 2 | 105382203 | R | F H L 2 |
| 1 0 9 | cg07082267 | GCTCCTCATGTGAGAAGGACCATAGGAATCTCCCGTT TCACAGGTGGGCACACCAAGGCC[CG]ACAATGGGTC CAGGCTGCCAAGGGTGGAGCCGAGATGCAAAGGGG CACCTCAGAGCCTGC | 1 6 | 83986536 | R | |
| 11 0 | cg07583137 | CAAACACCAGGGCAGCCCCATTTAAGGTTTTTGATAC ACTGAGGATCATTCAGAAAACTT[CG]GATTCCTAGTT ATAGAGTTGAATCCAACCACCAACACACTCCAGAAGT CCTGACATTAGG | 8 | 82806567 | R | C H M P 4 C |
| 111 | cg07547549 | TTGCAGCCTGGAGCTCAGCTCCATTGGAATGCTCCGG GCGCTGTCCAAGGTGCTGGAATG[CG]CCGCGCCCGG GGGCAGAGCTGCGGGCCGGGGGATTATCGCTGCCC ACGGCTTCGGGCTGA | 2 0 | 44091632 | R | S L C 1 2 A 5 |
| 11 2 | cg07553761 | AATCCGCATGGCACCGGTGGTCTGGGGGAGAGGCT GGGCCTGGCGCGGGACGAGGCGAAG[CG]CCGGTGG CCGACGGCTTCTGAGGAATTATCTTTTACTTGGCGCC ACACGGGGCGGGGCCT | 3 | 161650671 | F | T R I M 5 9 |
| 11 3 | cg25410668 | CTAGCCTCACAGCACCGCGTGGAGTTGCTTGTTCTTT TACATAGGAGGTCACATTCTCTT[CG]TGTAATGCCAC CAATGGTGCCGATTCTCCCCAGTGGGGCTGTGAGAA ACCTACGCCCTCT | 1 | 28114164 | R | R P A 2 |
| 11 4 | cg25478614 | TCGGGAGCTGAGGGACCCAGAAAAGCACCAAAACTC TTTAGAAGGACTGAGCATCCCTTA[CG]TCCAAACCAA TGGGGCAGGAGCAAGGCTTAGGGAGGGCTGGAGAA TCCGGGAGACGTCGA | 3 | 188870560 | R | SS T |
| 11 5 | cg22736354 | TGCGCCAGGGCGGCCACGCAGGCCAGGCAGACCAC GTGGCCGCAGGACAGGTTGCGCGGG[CG]CCGCTGC TGCCGGTGGCCAAACTTCTCAAAGCACACCTTGCACT CGAGCAGGCTGATCTC | 6 | 18230698 | F | N H L R C 1 |
| 11 6 | cg22454769 | TGCCCTCCGGGTCTTGGGAGCACAGTAGTTATCGGG AGCGTCGCCTCCGGCGTGGGCTCT[CG]GGCGCGAGT TTCGGACGAGGCCTGGGCGCGGTGGCAGGGGTCTG CCCACGCCGGGATCTC | 2 | 105382199 | R | F H L 2 |
| 11 7 | cg23500537 | CAGGAGTGCGGTGCAGCCACACATCCAAGGCTGACA GGGCGGGCACTCTGCCAAGTCCTG[CG]CGCTGCTCG CCTTCCACAACACCTTCCTCAGCTTCGTCTGTATTTGA AGAGCTTAGTAAA | 5 | 140400003 | R | |
| 11 8 | ch.2.304154 74F | ACCCGCTATCTGGTGGAGATAGAGAAAGGCAGATGG CTTGAAGATCCAATTTGGAGATTA[CA]TCCATAGGAC TTGCTGATGATCACATGTGGCAGAGGAAGAGCCAGT CCTCAAATATGGCC | 2 | 30415474 | F | |
| 11 9 | cg00748589 | CCGGTGCGCCGGGCTCTACCTCAAGGAGCTCAGGGC CATCGTGCTGAACCAACAGAGGCT[CG]TCCGCACCC AGCGCCAGAGCATCGACGAGCTGGAGCGGCGGCTG AACGAGCTGAGCGCCT | 1 2 | 11544753 | R | |
| 1 2 0 | cg23606718 | CTGACCGTGGTGCTGAGCGCGGCTCGCGCTCCGACG CGGTGCCCGAGCCTGTCGCGGCCG[CG]CCCTGCTGC ACTGCGGGCCCCCAGCGGTAAGTCGCCAAGGCCCCG AGAGGCTGCGTTGGT | 2 | 131230397 | F | F A M 1 2 3 C |
| 1 2 1 | cg21296230 | GGTGCGTTGTTCGCGGGGGTGAATTGTGAAGAACCA TCGCGGGGTCCTTCCTGCTGAGGC[CG]CGGACACCG TGACCTCGCTGCTCTGGGTCTGCAGGGAAACGTAGG AAAAAAAGTTGTCAG | 1 5 | 30797828 | R | G R E M 1 |
| 1 22 | cg03032497 | CACACCACTCGTATCTAACTCAACCCCTTTAGATATTC TTCCAGGTGGAATTATTGGATT[CG]GTCAGAATGGG GGAGGGGCCACTATGCCCTTAAGAGGCTCAGAAGTG CCTACCTGGCTAA | 1 4 | 60177980 | R | |
| 1 2 3 | cg18473521 | CTCCTCGGCCCCCTCTGGCCCCCGTCCTCCTTTCTCTC CTTCCCCCTCTCTCTCCAGGAG[CG]ACTCTGGGTTAG CACAATTGAACTGGATTTACGAGCGAGAATGGGTAA TTACATCCCCCA | 1 2 | 52734532 | F | H O X C 4 |
| 1 2 4 | cg06639320 | CCTTTGTTTGCCAGGGCTCCTTTCTTCGTGCCCTCCGG GTCTTGGGAGCACAGTAGTTAT[CG]GGAGCGTCGCC TCCGGCGTGGGCTCTCGGGCGCGAGTTTCGGACGAG GCCTGGGCGCGGT | 2 | 105382171 | R | F H L 2 |
| 1 2 5 | cg08540945 | CCCCGAGGCGGACGCCAGAGGGCGCGCCCCCCAC TCCTGCCCGCGTCGGGCCGCAGC[CG]CGCTCCGCC CTTTGCCTGCAGAGCGCTGGGGTTTAAAGTCCTGA ACCCATGCACGGCTG | 7 | 152222631 | F | |
| 1 2 6 | cg06493994 | GGAGAGCAAGTCAAGAAATACGGTGAAGGAGTCCTT CCCAAAGTTGTCTAGGTCCTTCCG[CG]CCGGTGCCTG GTCTTCGTCGTCAACACCATGGACAGCTCCCGGGAAC CGACTCTGGGGCG | 6 | 25760581 | R | S C G N |
| 1 2 7 | cg04400972 | GGGTATTTTTCGGGGGTGAGGGCATCTCAGGCTCGC GTTACGGGGACCGGTTCGGGAGAC[CG]TGGAGCCG AGGTGTCGAATGGAGGGCTTACTTCGGCCGGGCCAG GGAGCGCCACCCTCCT | 1 | 117466576 | R | T R I M 4 5 |
| 1 2 8 | cg02650266 | GCCCGAGAGGATCCAGGGAAAGCAGAAGGGGGTTA AGGACCATGGACAGAGCCCGTCGCG[CG]CTCGTTGC TGCCGCCTTCCCCAGCACTCTGGCGGCTCCTGAGGAC AGCGGTCCCATCTTG | 4 | 147777689 | F | |
| 1 2 9 | cg03607117 | CGCTGTGGCCCCGAGCGGAACGGCCCGGAAGAGGA GACGCGTCCCCGGGAACCCAGTGCC[CG]CCCTGGCC CAGCCCCGATCCAGCCTGCGCCTCACCTCGGGTTGTA GACAGAGCGGCGGGG | 3 | 53055480 | F | S F M B T 1 |
| 1 3 0 | cg14361627 | TCGGAACGCGCGGCCCCCCGGCTAAGTCATGTTTAA CAGCCTCAGAAATTATCTTGTCTC[CG]CGTTCTTTCTT CTGCCGGCGAGCCAGGTAATGGTAACAGAGCGAAAC TCCCCAGTCGGAA | 7 | 130069656 | R | K L F 1 4 |
| 1 3 1 | cg16867657 | CCGCGGCGTCCCCTGCCGGCCGGGCGGCGATTTGCA GGTCCAGCCGGCGCCGGTTTCGCG[CG]GCGGCTCAA CGTCCACGGAGCCCCAGGAATACCCACCCGCTGCCC AGATCGGCAGCCGCT | 6 | 11152863 | F | E L O V L 2 |
| 1 3 2 | cg04940570 | GCGCACACACGCACACACCCTCGGGCGCCTTGGACG GGGTGCGCTGGGGAGCCAGAAGTT[CG]GAGCGAGC GCGGGCGGGCAGAGCCGCCGCCTCGGAGCCCGGAG CCGGCCTGCACCCCCCT | 11 | 12653334 | R | T E A D 1 |
| 1 33 | cg04416734 | ACTGTCTCTGCTTCGAGATCAAGCTCCGATGAGGACC CAGGGCCCCTGCCCTCTGGGGAG[CG]GCCAGCCCCC AGGGCCCATGTGCCCTCCTCCCTGAAGAGCCTTTCCC CACGCCACTGGAA | 1 6 | 29982693 | R | A L D O A |
| 1 3 4 | cg06419846 | CACCGGGCTCACACTGCTGCTCGCACGGAGCCTGGG CACAGGGGTCCTCGCAACTGCGCC[CG]TCTGCTGCC AGCCGGAAGCCCTCAGTGCAGCGGCAGGACACGTG ACCATCCACCTCCTCC | 11 | 65840273 | F | C D 2 4 8 |
| 1 3 5 | cg19935065 | GCAACACAGAGTGCTTTACCCATGCCTTAAGGCAGCT GTCACATGACTCTTCCCCTAATA[CG]GTTGTGAGGTG CTCACGTGTTTTGGAGATAGCAAAAGTCTCAAATAAT ATGGCACAGACG | 1 0 | 98052677 | F | D N T T |
| 1 3 6 | cg07955995 | AGAAATTATCTTGTCTCCGCGTTCTTTCTTCTGCCGGC GAGCCAGGTAATGGTAACAGAG[CG]AAACTCCCCAG TCGGAACTTCTGGGTTGCAGCAGCCTCGCCCCCCCTC CCCGCAGCCCGC | 7 | 130069699 | R | K L F 1 4 |
| 1 3 7 | cg11067179 | CCTCGCTACAATAACACTCGAAGCCACCAACGTAGTT GACACACATCTGCTGGCACACAC[CG]GCAATCTGGC ACTCATCTGTGTCCACACAGCGGTGCGGATCATCCTC CGCTGGCCGGAAA | 11 | 65840117 | F | C D 2 4 8 |
| 1 3 8 | cg21139312 | TTGGAGCTCGCCAAAGCCGCCCCGGGCTCTGTGGTTT CCAGAGCAGATGCGGAGGCGGCA[CG]TCCTCGTGCC CTTGCTCCAGCTGCGCACACGACCTCAGCCTCCTCTG CCCCGCTGGGCGC | 1 7 | 53018224 | R | M S I 2 |
| 1 3 9 | cg14692377 | ACCCACCTGCCAGGCTGCGCGGGGAGGCTGGTCCCG GGCTGGGCAGGCGGGCTGGCCTCG[CG]CCCTCGAG GCACCCGGCGGCGCTGGCTGTGCGGAGGGGCGCCG GCGCGGCCGTATTTGTA | 1 7 | 25586811 | R | S L C 6 A 4 |
| 1 4 0 | cg20426994 | GAAGGGGCATTGGTGGCGCTTGGCAGCAGGTGTGA CAGACCTCCTCCGGGGCGCCTGATC[CG]CGGCGGGG GCGGGGCCTGCCCCTAGGGCCCCTCCAGAGAACCCA CCAGAGGCTGCTGGTG | 7 | 130068864 | R | K L F 1 4 |
| 1 4 1 | cg22213242 | TAGTTGACACACATCTGCTGGCACACACCGGCAATCT GGCACTCATCTGTGTCCACACAG[CG]GTGCGGATCA TCCTCCGCTGGCCGGAAACCCAGGCGACAGTGGCAG CTGTAGCCTTGTGG | 11 | 65840149 | R | C D 2 4 8 |
| 1 4 2 | cg08097417 | CCGGCTAAGTCATGTTTAACAGCCTCAGAAATTATCT TGTCTCCGCGTTCTTTCTTCTGC[CG]GCGAGCCAGGT AATGGTAACAGAGCGAAACTCCCCAGTCGGAACTTC TGGGTTGCAGCAG | 7 | 130069673 | R | K L F 1 4 |
| 1 4 3 | cg03399905 | TTCACCTGACCCCCATGCTCGGCCTTCTGGAAGATGC CCACAGACACTGGCAATAATGGA[CG]CTGGCACACT CTGCCGGCGCGGCCGGAGCCCCGGAGTTCAGCACCT CGGACAGAGCCCGA | 1 5 | 77363115 | R | A N K R D 3 4 C |

## Claims

1. A method of estimating the epigenetic age of an individual, the method comprising:
observing a presence or absence of methyl groups at a plurality of CpG markers that correlate with:
counts of naive cytotoxic T cells in the population of leukocytes obtained from the individual;
counts of exhausted cytotoxic T cells in the population of leukocytes obtained from the individual; and
counts of plasma B cells in the population of leukocytes obtained from the individual; and
comparing the methylation status of the plurality of CpG markers observed in the population of leukocytes from the individual with the methylation status of the same markers from an age matched reference population so as to obtain a value or a range of values for cell counts of naive cytotoxic T cells, exhausted cytotoxic T cells, and plasma B cells; and
estimating the epigenetic age of the individual,
wherein
the plurality of CpG markers that correlate with counts of naive cytotoxic T cells comprises cg 15867698, cg 17478979, cg25289028, cg10909506, cg23001918, cg17820878, cg07107916, cg09025210, cg07899551, cg03370106, cg26485825, cg21097090, cg25130381, cgl6662477, cg06952412, cg05392293, cg26720010, cg04683740, cg08945443, cg20386303, cg15535471, cg25020550, cg24376214, cg21593149, cg25639084, cg15989436, cg02033323, cg18346531, cg02989940, cg10274029, cg07955474, cgl8442362, cg23876292, cg12966876, cg17850367, cg01372366, cg05913271, cg07094298 and cg10493055;
the plurality of CpG markers that correlate with counts of exhausted cytotoxic T cells comprises cg07094298, cg04683740, cg26655856, cg14518178, cg01372366, cg09197075, cg03132824,
cg00147638, cg25020550, cg23731272, cg22513455, cg00495443, cg08688907, cg21912203, cg10688297, cg10909506, cg26091609, cg00073460, cg08454507, cg20723792, cg14969094, cg01124420, cg06445016, cg05217983, cg03467087, cg02988775, cg01345395, cg16549957, cg21593149, cg00871371, cg11685391, cg23001918, cg26485825, cg08482359 and cg13608166; and;
the plurality of CpG markers that correlate with counts of plasma B cells comprises cg24735235, cg01372366, cg25521400, cg01124420, cg13553498, cg26164712, cg20244489, cg08945443, cg13608166 and cg06245711.

2. The method of claim 1, wherein the epigenetic age of the individual is estimated using a weighted average of DNA methylation levels.

3. The method of claim 1 or 2, wherein the method further comprises obtaining the chronological age of the individual and comparing the chronological age with the estimated epigenetic age so as to observe a presence or absence of epigenetic age acceleration in the individual.

4. The method of claims 1-3, wherein observing the presence or absence of methyl groups at a plurality of CpG markers comprises treatment of genomic DNA from the population of leukocytes with bisulfite to transform unmethylated cytosines of CpG dinucleotides in the genomic DNA to uracil.

5. The method of claims 1-4, wherein the population of leukocytes is obtained from blood or saliva of the individual.

6. The method of claim 3, wherein an estimated epigenetic age that is greater than the true chronological age of the individual correlates an increased risk of all-cause mortality in the individual.

7. The method of any one of claim 1-6, wherein the population of leukocytes is obtained from saliva of the individual.

8. A tangible computer-readable medium comprising computer-readable code that, when executed by a computer, causes the computer to perform operations comprising:
a) receiving information corresponding to methylation levels of a set of methylation markers in a biological sample;
b) determining an epigenetic age by applying a statistical prediction algorithm to the set of methylation markers;
c) determining an epigenetic age based on a weighted average of the methylation levels for cell counts of naive cytotoxic T cells, exhausted cytotoxic T cells and plasma B cells; and
d) comparing the determined epigenetic age to a chronological age of the biological sample,
wherein
the set of methylation markers that correlate with counts of naive cytotoxic T cells comprises cg 15867698, cg 17478979, cg25289028, cg10909506, cg23001918, cg17820878, cg07107916, cg09025210, cg07899551, cg03370106, cg26485825, cg21097090, cg25130381, cgl6662477, cg06952412, cg05392293, cg26720010, cg04683740, cg08945443, cg20386303, cg15535471, cg25020550, cg24376214, cg21593149, cg25639084, cg15989436, cg02033323, cg18346531, cg02989940, cg10274029, cg07955474, cgl8442362, cg23876292, cg12966876, cg17850367, cg01372366, cg05913271, cg07094298 and cg10493055;
the set of methylation markers that correlate with counts of exhausted cytotoxic T cells comprises cg07094298, cg04683740, cg26655856, cg14518178, cg01372366, cg09197075, cg03132824, cg00147638, cg25020550, cg23731272, cg22513455, cg00495443, cg08688907, cg21912203, cg10688297, cg10909506, cg26091609, cg00073460, cg08454507, cg20723792, cg14969094, cg01124420, cg06445016, cg05217983, cg03467087, cg02988775, cg01345395, cg16549957, cg21593149, cg00871371, cg11685391, cg23001918, cg26485825, cg08482359 and cg13608166; and;
the set of methylation markers that correlate with counts of plasma B cells comprises cg24735235, cg01372366, cg25521400, cg01124420, cg13553498, cg26164712, cg20244489, cg08945443, cg13608166 and cg06245711

9. The tangible computer-readable medium of claim 8, wherein the step of receiving information comprises receiving from a tangible data storage device information corresponding to the methylation levels of the set of methylation markers in the biological sample.

## Patentansprüche

1. Verfahren zur Schätzung des epigenetischen Alters eines Individuums, wobei das Verfahren Folgendes umfasst:
Beobachten der Anwesenheit oder Abwesenheit von Methylgruppen an einer Vielzahl von CpG-Markern, die mit Folgendem korrelieren:
der Anzahl der naiven zytotoxischen T-Zellen in der Leukozytenpopulation des Individuums;
der Anzahl der erschöpften zytotoxischen T-Zellen in der Leukozytenpopulation des Individuums; und
der Anzahl von Plasma-B-Zellen in der Population von Leukozyten, die von dem Individuum erhalten wurden; und
Vergleichen des Methylierungsstatus der Vielzahl von CpG-Markern, die in der Leukozytenpopulation des Individuums beobachtet wurden, mit dem Methylierungsstatus der gleichen Marker aus einer altersgleichen Referenzpopulation, um einen Wert oder einen Bereich von Werten für die Zellzahlen von naiven zytotoxischen T-Zellen, erschöpften zytotoxischen T-Zellen und Plasma-B-Zellen zu erhalten; und
Schätzen des epigenetischen Alters des Individuums,
wobei
die Vielzahl von CpG-Markern, die mit der Anzahl von naiven zytotoxischen T-Zellen korrelieren, Folgendes umfasst:
cg 15867698, cg 17478979, cg25289028, cg10909506, cg23001918, cg17820878, cg07107916, cg09025210, cg07899551, cg03370106, cg26485825, cg21097090, cg25130381, cgl6662477, cg06952412, cg05392293, cg26720010, cg04683740, cg08945443, cg20386303, cg15535471, cg25020550, cg24376214, cg21593149, cg25639084, cg15989436, cg02033323, cg18346531, cg02989940, cg10274029, cg07955474, cgl8442362, cg23876292, cg12966876, cg17850367, cg01372366, cg05913271, cg07094298 und cg10493055;
die Vielzahl der CpG-Marker, die mit der Anzahl erschöpfter zytotoxischer T-Zellen korrelieren, Folgendes umfasst:
cg07094298, cg04683740, cg26655856, cg14518178, cg01372366, cg09197075, cg03132824,
cg00147638, cg25020550, cg23731272, cg22513455, cg00495443, cg08688907, cg21912203, cg10688297, cg10909506, cg26091609, cg00073460, cg08454507, cg20723792, cg14969094, cg01124420, cg06445016, cg05217983, cg03467087,
cg02988775, cg01345395, cg16549957, cg21593149, cg00871371, cg11685391, cg23001918, cg26485825, cg08482359 und cg13608156 ; und
die Vielzahl der CpG-Marker, die mit der Anzahl der Plasma-B-Zellen korrelieren, Folgendes umfasst:
cg24735235, cg01372366, cg25521400, cg01124420, cg13553498, cg26164712, cg20244489, cg08945443, cg136Q816δ und cg06245711.

2. Verfahren nach Anspruch 1, wobei das epigenetische Alter des Individuums unter Verwendung eines gewichteten Durchschnitts der DNA-Methylierungsniveaus geschätzt wird.

3. Verfahren nach Anspruch 1 oder 2, wobei das Verfahren ferner umfasst, das chronologische Alter des Individuums zu ermitteln und das chronologische Alter mit dem geschätzten epigenetischen Alter zu vergleichen, um das Vorhandensein oder die Abwesenheit einer epigenetischen Altersbeschleunigung bei dem Individuum zu beobachten.

4. Verfahren nach Anspruch 1-3, wobei das Beobachten des Vorhandenseins oder der Abwesenheit von Methylgruppen an einer Vielzahl von CpG-Markern die Behandlung von genomischer DNA aus der Population von Leukozyten mit Bisulfit umfasst, um unmethylierte Cytosine von CpG-Dinukleotiden in der genomischen DNA in Uracil umzuwandeln.

5. Verfahren nach Anspruch 1-4, wobei die Population von Leukozyten aus Blut oder Speichel des Individuums gewonnen wird.

6. Verfahren nach Anspruch 3, wobei ein geschätztes epigenetisches Alter, das höher ist als das tatsächliche chronologische Alter des Individuums, mit einem erhöhten Risiko der Gesamtmortalität des Individuums korreliert.

7. Verfahren nach einem der Ansprüche 1-6, wobei die Population von Leukozyten aus dem Speichel des Individuums gewonnen wird.

8. Greifbares computerlesbares Medium, das computerlesbaren Code umfasst, der, wenn er von einem Computer ausgeführt wird, den Computer veranlasst, Operationen durchzuführen, die Folgendes umfassen:
a) Empfangen von Informationen, die den Methylierungsniveaus eines Satzes von Methylierungsmarkern in einer biologischen Probe entsprechen;
b) Bestimmen eines epigenetischen Alters durch Anwendung eines statistischen Vorhersagealgorithmus auf den Satz von Methylierungsmarkern;
c) Bestimmen eines epigenetischen Alters basierend auf einem gewichteten Durchschnitt der Methylierungsniveaus für Zellzahlen von naiven zytotoxischen T-Zellen, erschöpften zytotoxischen T-Zellen und Plasma-B-Zellen; und
d) Vergleichen des bestimmten epigenetischen Alters mit einem chronologischen Alter der biologischen Probe,
wobei
der Satz von Methylierungsmarkern, der mit der Anzahl naiver zytotoxischer T-Zellen korreliert, Folgendes umfasst
cg 15867698, cg 17478979, cg25289028, cg10909506, cg23001918, cg17820878, cg07107916, cg09025210, cg07899551, cg03370106, cg26485825, cg21097090, cg25130381, cg16662477, cg06952412, cg05392293, cg26720010, cg04683740, cg08945443, cg20386303, cg15535471, cg25020550, cg24376214, cg21593149, cg25639084, cg15989436, cg02033323, cg18346531, cg02989940, cg10274029, cg07955474, cg184423δ2, cg23876292, cg1296δ87δ, cg17850367, cg01372366, cg05913271, cg07094298 und cg10493055;
der Satz von Methylierungsmarkern, der mit der Anzahl von erschöpften zytotoxischen T-Zellen korreliert, Folgendes umfasst:
cg07094298, cg04683740, cg26655856, cg14518178, cg01372366, cg09197075, cg03132824, cg00147638, cg25020550, cg23731272, cg22513455, cg00495443, cg08688907, cg21912203, cg10688297, cg10909506, cg2δ091609, cg00073460, cg08454507, cg20723792, cg14969094, cg01124420, cg06445016, cg05217983, cg03467087, cg02988775, cg01345395, cg16549957, cg21593149, cg00871371, cg11685391, cg23001918, cg26485825, cg08482359 und cg13608166; und
der Satz von Methylierungsmarkern, der mit der Anzahl von Plasma-B-Zellen korreliert, Folgendes umfasst:
cg24735235, cg01372366, cg25521400, cg01124420, cg13553498, cg26164712, cg20244489, cg08945443, cg13608166 und cg06245711

9. Greifbares computerlesbares Medium nach Anspruch 8, wobei der Schritt des Empfangens von Informationen das Empfangen von Informationen, die den Methylierungsniveaus des Satzes von Methylierungsmarkern in der biologischen Probe entsprechen, von einer greifbaren Datenspeichereinrichtung umfasst.

## Revendications

1. Méthode d'estimation de l'âge épigénétique d'un individu, la méthode comprenant :
l'observation d'une présence ou absence de groupes méthyle à une pluralité de marqueurs CpG qui se corrèlent avec :
des comptes de cellules T cytotoxiques naïves dans la population de leucocytes obtenus de l'individu ;
des comptes de cellules T cytotoxiques épuisées dans la population de leucocytes obtenus de l'individu ; et
des comptes de cellules B plasmatiques dans la population de leucocytes obtenus de l'individu ; et
la comparaison de l'état de méthylation de la pluralité de marqueurs CpG observés dans la population de leucocytes provenant de l'individu à l'état de méthylation des mêmes marqueurs provenant d'une population de référence assortie en âge afin d'obtenir une valeur ou une plage de valeurs pour des comptes cellulaires de cellules T cytotoxiques naïves, de cellules T cytotoxiques épuisées, et de cellules B plasmatiques ; et
l'estimation de l'âge épigénétique de l'individu,
dans laquelle
la pluralité de marqueurs CpG qui se corrèlent avec des comptes de cellules T cytotoxiques naïves comprend cg15867698, cg17478979, cg25289028, cg10909506, cg23001918, cg17820878, cg07107916, cg09025210, cg07899551, cg03370106, cg26485825, cg21097090, cg25130381, cg16662477, cg06952412, cg05392293, cg26720010, cg04683740, cg08945443, cg20386303, cg15535471, cg25020550, cg24376214, cg21593149, cg25639084, cg15989436, cg02033323, cg18346531, cg02989940, cg10274029, cg07955474, cg18442362, cg23876292, cg12966876, cg17850367, cg01372366, cg05913271, cg07094298 et cg10493055 ;
la pluralité de marqueurs CpG qui se corrèlent avec des comptes de cellules T cytotoxiques épuisées comprend cg07094298, cg04683740, cg26655856, cg14518178, cg01372366, cg09197075, cg03132824, cg00147638, cg25020550, cg23731272, cg22513455, cg00495443, cg08688907, cg21912203, cg10688297, cg10909506, cg26091609, cg00073460, cg08454507, cg20723792, cg14969094, cg01124420, cg06445016, cg05217983, cg03467087, cg02988775, cg01345395, cg16549957, cg21593149, cg00871371, cg11685391, cg23001918, cg26485825, cg08482359 et cg13608166 ; et ;
la pluralité de marqueurs CpG qui se corrèlent avec des comptes de cellules B plasmatiques comprend cg24735235, cg01372366, cg25521400, cg01124420, cg13553498, cg26164712, cg20244489, cg08945443, cg13608166 et cg06245711.

2. Méthode de la revendication 1, dans laquelle l'âge épigénétique de l'individu est estimé en utilisant une moyenne pondérée de niveaux de méthylation d'ADN.

3. Méthode de la revendication 1 ou 2, dans laquelle la méthode comprend en outre l'obtention de l'âge chronologique de l'individu et la comparaison de l'âge chronologique à l'âge épigénétique estimé afin d'observer une présence ou absence d'accélération d'âge épigénétique chez l'individu.

4. Méthode des revendications 1 à 3, dans laquelle l'observation de la présence ou de l'absence de groupes méthyle à une pluralité de marqueurs CpG comprend le traitement d'ADN génomique provenant de la population de leucocytes avec du bisulfite pour transformer des cytosines non méthylées de dinucléotides CpG dans l'ADN génomique en uracile.

5. Méthode des revendications 1 à 4, dans laquelle la population de leucocytes est obtenue de sang ou de salive de l'individu.

6. Méthode de la revendication 3, dans laquelle un âge épigénétique estimé qui est supérieur à l'âge chronologique vrai de l'individu corrèle un risque accru de mortalité de toute cause chez l'individu.

7. Méthode d'une quelconque de la revendication 1 à 6, dans laquelle la population de leucocytes est obtenue de salive de l'individu.

8. Support matériel lisible par ordinateur comprenant un code lisible par ordinateur qui, lorsqu'il est exécuté par un ordinateur, amène l'ordinateur à réaliser des opérations comprenant :
a) la réception d'informations correspondant à des niveaux de méthylation d'un ensemble de marqueurs de méthylation dans un échantillon biologique ;
b) la détermination d'un âge épigénétique en appliquant un algorithme de prédiction statistique à l'ensemble de marqueurs de méthylation ;
c) la détermination d'un âge épigénétique sur la base d'une moyenne pondérée des niveaux de méthylation pour des comptes cellulaires de cellules T cytotoxiques naïves, de cellules T cytotoxiques épuisées et de cellules B plasmatiques ; et
d) la comparaison de l'âge épigénétique déterminé à un âge chronologique de l'échantillon biologique,
dans lequel
l'ensemble de marqueurs de méthylation qui se corrèlent avec des comptes de cellules T cytotoxiques naïves comprend cg15867698, cg17478979, cg25289028, cg10909506, cg23001918, cg17820878, cg07107916, cg09025210, cg07899551, cg03370106, cg26485825, cg21097090, cg25130381, cg16662477, cg06952412, cg05392293, cg26720010, cg04683740, cg08945443, cg20386303, cg15535471, cg25020550, cg24376214, cg21593149, cg25639084, cg15989436, cg02033323, cg18346531, cg02989940, cg10274029, cg07955474, cg18442362, cg23876292, cg12966876, cg17850367, cg01372366, cg05913271, cg07094298 et cg10493055 ;
l'ensemble de marqueurs de méthylation qui se corrèlent avec des comptes de cellules T cytotoxiques épuisées comprend cg07094298, cg04683740, cg26655856, cg14518178, cg01372366, cg09197075, cg03132824, cg00147638, cg25020550, cg23731272, cg22513455, cg00495443, cg08688907, cg21912203, cg10688297, cg10909506, cg26091609, cg00073460, cg08454507, cg20723792, cg14969094, cg01124420, cg06445016, cg05217983, cg03467087, cg02988775, cg01345395, cg16549957, cg21593149, cg00871371, cg11685391, cg23001918, cg26485825, cg08482359 et cg13608166 ; et
l'ensemble de marqueurs de méthylation qui se corrèlent avec des comptes de cellules B plasmatiques comprend cg24735235, cg01372366, cg25521400, cg01124420, cg13553498, cg26164712, cg20244489, cg08945443, cg13608166 et cg06245711.

9. Support matériel lisible par ordinateur de la revendication 8, dans lequel l'étape de la réception d'informations comprend la réception, en provenance d'un dispositif matériel de stockage de données, d'informations correspondant aux niveaux de méthylation de l'ensemble de marqueurs de méthylation dans l'échantillon biologique.
